# EUROPÄISCHE PATENTSCHRIFT

(11) **EP 3 872 157 B1**
(45) Veröffentlichungstag und Bekanntmachung des Hinweises auf die Patenterteilung: **28.12.2022**
(21) Anmeldenummer: 21157270.6
(22) Anmeldetag: 16.02.2021
(51) Int. Cl.: C11D 3/34, C11D 11/00, C07D 257/00, C07D 257/02, C07F 5/06

(54) **GESCHIRRSPÜLMITTEL ENTHALTEND METALLKOMPLEXE**
DISHWASHING COMPOSITIONS CONTAINING METAL COMPLEXES
PRODUIT DE LAVAGE POUR VAISSELLE LES CONTENANT DES COMPLEXES MÉTALLIQUES

(30) Priorität: 27.02.2020 DE 102020202492; 24.06.2020 DE 102020207791
(43) Veröffentlichungstag der Anmeldung: 01.09.2021
(73) Patentinhaber: Henkel AG & Co. KGaA, 40589 Düsseldorf (DE)
(72) Erfinder: Kropf, Christian, 40724 Hilden (DE); Polarz, Sebastian, 30900 Wedemark (DE); Frisch, Marvin Lionel, 10719 Berlin (DE)

(56) Entgegenhaltungen:
- WO-A1-2016/062784
- CHANG JOHN YU-CHIH ET AL: "Syntheses of 8-quinolinolatocobalt(iii) complexes containing cyclen based auxiliary ligands as models for hypoxia-activated prodrugs", DALTON TRANSACTIONS, Bd. 39, Nr. 48, 28. Dezember 2010 (2010-12-28), Seiten 11535-11550, XP055789232, Cambridge ISSN: 1477-9226, DOI: 10.1039/c0dt01142h Gefunden im Internet: URL:https://pubs.rsc.org/en/content/articl epdf/2010/dt/c0dt01142h>
- LACERDA SARA ET AL: "A novel tetraazamacrocycle bearing a thiol pendant arm for labeling biomolecules with radiolanthanides", DALTON TRANSACTIONS, Nr. 23, 9. April 2009 (2009-04-09), Seite 4509, XP055814873, Cambridge ISSN: 1477-9226, DOI: 10.1039/b820375j Gefunden im Internet: URL:https://pubs.rsc.org/en/content/articl epdf/2009/dt/b820375j>
- LACERDA SARA ET AL: "Study of the cyclen derivative 2-[1,4,7,10-tetraazacyclododecan-1-yl]-eth anethiol and its complexation behaviour towards d-transition metal ions", POLYHEDRON, Bd. 26, Nr. 14, 1. August 2007 (2007-08-01), Seiten 3763-3773, XP055815139, GB ISSN: 0277-5387, DOI: 10.1016/j.poly.2007.04.037

## Beschreibung

Die vorliegende Erfindung betrifft ein maschinelles Geschirrspülmittel, das durch den Gehalt an Metallkomplexen eine verbesserte Reinigungsleistung beim Entfernen von eingebrannten Anschmutzungen zeigt, die Verwendung dieses Geschirrspülmittels sowie ein Verfahren zum maschinellen Geschirrspülen unter Einsatz dieses Geschirrspülmittels.

Das wichtigste Kriterium beim maschinellen Geschirrspülen ist die Reinigungsleistung an verschiedensten Anschmutzungen, welche oft in Form von Lebensmittelresten in die Geschirrspülmaschine eingebracht werden. Gerade bei hartnäckigen Anschmutzungen, wie zum Beispiel solchen, die bei der Zubereitung von protein- und stärkehaltigen Lebensmitteln bei hohen Temperaturen (Braten, Backen, Frittieren, Gratinieren, etc.) entstehen, sogenanntem eingebranntem Schmutz, ist die Reinigungsleistung verfügbarer Geschirrspülmittel nach wie vor nicht zufriedenstellend. Eine unzureichende Reinigungsleistung führen beim Verbraucher zu Unzufriedenheit. Daher besteht ein allgemeiner Bedarf nach maschinellen Geschirrspülmitteln, die auch bei eingebrannten Anschmutzungen noch eine gute Reinigungsleistung aufweisen.

Aus der internationalen Patentanmeldung WO 2016/062784 A1 sind reinigungsverstärkende N-substituierte 1,4,7,10-Tetraazacyclododecane bekannt.

Es wurde nun überraschenderweise gefunden, dass Metallkomplexe mit bestimmten heterocyclischen Liganden, die Sulfo- oder Sulfato- oder Thioalkylsubstituenten am Heteroatom tragen, bei der Verwendung in Geschirrspülen eine verbesserte Reinigungsleistung an eingebrannten Anschmutzungen bewirken.

Ein erster Aspekt betrifft eine Verbindung der allgemeinen Formel (I) 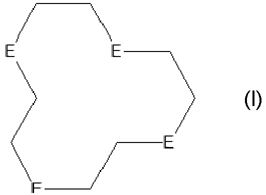 in der jedes E unabhängig voneinander für O oder NR¹ steht mit der Maßgabe, dass mindestens 1 E nicht O ist, jedes R¹ unabhängig voneinander für H, unsubstituiertes oder substituiertes, lineares oder verzweigtes C₁₋₂₂ Alkyl, unsubstituiertes oder substituiertes, lineares oder verzweigtes C₁₋₂₂ Heteroalkyl, unsubstituiertes oder substituiertes Aryl, unsubstituiertes oder substituiertes Heteroaryl, unsubstituiertes oder substituiertes, lineares oder verzweigtes C₂₋₂₀ Alkenyl, unsubstituiertes oder substituiertes, lineares oder verzweigtes C₂₋₂₀ Alkinyl, unsubstituiertes oder substituiertes, lineares oder verzweigtes C₂₋₂₀ Heteroalkenyl, unsubstituiertes oder substituiertes, lineares oder verzweigtes Alkylaryl, oder unsubstituiertes oder substituiertes, lineares oder verzweigtes Alkylheteroaryl steht mit der Maßgabe, dass mindestens 1 R¹ für ein substituiertes C₁₋₂₂ Alkyl der allgemeinen Formel (IIa) oder (IIb) steht, oder mit der Maßgabe, dass mindestens 1 R¹ für ein substituiertes C₁₋₂₂ Alkyl der allgemeinen Formel (IIIa) oder (IIIb) steht, 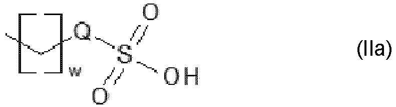 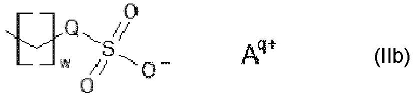 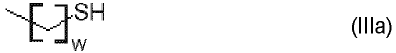 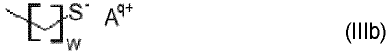 in denen Q für O oder CH₂, w für eine Zahl von 1 bis 22, q für 1 oder 2 und A⁺ für ein Kation, ausgewählt aus Alkalimetallkationen mit q = 1, ½Erdalkalikationen mit q = 2 und Ammoniumionen mit q = 1, steht.

Vorzugsweise sind 1, insbesondere 2, besonders bevorzugt 3 oder 4 E nicht O. Vorzugsweise steht w für eine Zahl von 2 bis 20, insbesondere von 3 bis 18.

Vorzugsweise entspricht in der allgemeinen Formel (I) 1 Gruppe R¹ der allgemeinen Formel (IIa), (IIb), (IIIa) oder (IIIb), wobei Mischungen aus Verbindungen mit R¹ = IIa und R¹ = IIb sowie Mischungen aus Verbindungen mit R¹ = IIIa und R¹ = IIIb zwanglos möglich sind und der teilweisen Salzbildung aus Verbindungen mit R¹ = IIa und R¹ = IIIa entsprechen. Die Gruppen R¹, die nicht der allgemeinen Formel (IIa), (IIb), (IIIa) oder (IIIb) entsprechen, werden vorzugsweise aus 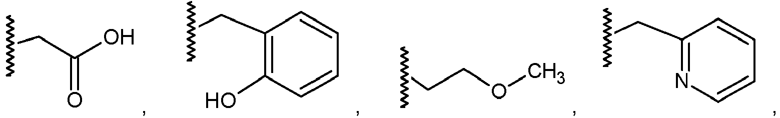 C₁₋₁₀ Alkyl, 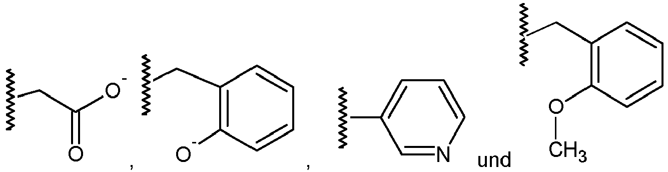 ausgewählt, wobei die gezeigten Anionen durch die Anwesenheit eines oben definierten Kations A^{q+} zur Ladungsneutralität gebracht werden.

Ein weiterer Gegenstand ist ein Metallkomplex der der allgemeinen Formel (III)

(A^{q+})ₚ[Mⁿ⁺L^{m-}](X^{o-})ᵣ (III)

in der q für 1 oder 2 und A⁺ für ein Kation, ausgewählt aus Alkalimetallkationen mit q = 1, ½Erdalkalikationen mit q = 2 und Ammoniumionen mit q = 1, steht, Mⁿ⁺ für ein Aluminiumion, ein Übergangsmetallion oder ein Lanthanoidmetallion steht, L für einen Liganden der Formel (I) 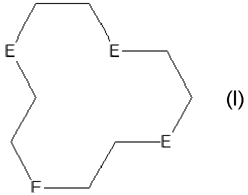 in der jedes E unabhängig voneinander für O oder NR¹ steht mit der Maßgabe, dass mindestens 1 E nicht O ist, jedes R¹ unabhängig voneinander für H, unsubstituiertes oder substituiertes, lineares oder verzweigtes C₁₋₂₂ Alkyl, unsubstituiertes oder substituiertes, lineares oder verzweigtes C₁₋₂₂ Heteroalkyl, unsubstituiertes oder substituiertes Aryl, unsubstituiertes oder substituiertes Heteroaryl, unsubstituiertes oder substituiertes, lineares oder verzweigtes C₂₋₂₀ Alkenyl, unsubstituiertes oder substituiertes, lineares oder verzweigtes C₂₋₂₀ Alkinyl, unsubstituiertes oder substituiertes, lineares oder verzweigtes C₂₋₂₀ Heteroalkenyl, unsubstituiertes oder substituiertes, lineares oder verzweigtes Alkylaryl, oder unsubstituiertes oder substituiertes, lineares oder verzweigtes Alkylheteroaryl steht mit der Maßgabe, dass mindestens 1 R¹ für ein substituiertes C₁₋₂₂ Alkyl der allgemeinen Formel (IIa) oder (llc) steht, oder mit der Maßgabe, dass mindestens 1 R¹ für ein substituiertes C₁₋₂₂ Alkyl der allgemeinen Formel (IIIa) oder (IIIc) steht, 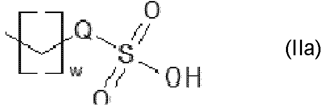 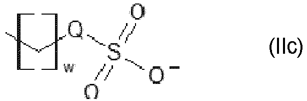 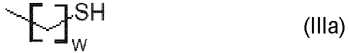 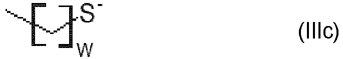 in denen Q für O oder CH₂, w für eine Zahl von 1 bis 22, q für 1 oder 2 und A⁺ für ein Kation, ausgewählt aus Alkalimetallkationen mit q = 1, ½Erdalkalikationen mit q = 2 und Ammoniumionen mit q = 1, steht, X^{o-} für ein Anion, ausgewählt aus F⁻, Cl⁻, Br⁻, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, Acetat, Citrat, Formiat, Glutarat, Lactat, Malat, Malonat, Oxalat, Pyruvat, Tartrat, Methansulfonat, Methylsulfat, p-Toluolsulfat und Succinat, steht, n für eine Zahl von 1 bis 5 steht, m für eine Zahl von 0 bis 4 und o für eine Zahl von 1 bis 3 steht sowie p und r unabhängig voneinander für eine Zahl von 0 und 7 stehen mit der Maßgabe, dass die Summe aus n und dem Produkt aus p und q gleich der Summe aus m und dem Produkt aus r und o ist.

Bekanntlich kommt nicht jede Zahl aus dem für n definierten Bereich für jedes Metall in Frage, weil Metalle der Gruppe 3 des Periodensystems der Elemente normalerweise in der Oxidationsstufe +3, Metalle der Gruppe 4, Gruppe 7, Gruppe 8, Gruppe 9, Gruppe 10 und die Lanthanoidmetalle in den Oxidationsstufen +2, +3 oder +4, Metalle der Gruppe 5 in den Oxidationsstufen +2, +3, +4 oder +5, Metalle der Gruppe 6 in den Oxidationsstufen +2 oder +3, Metalle der Gruppe 11 in den Oxidationsstufen +1, +2 oder +3, Metalle der Gruppe 12 in den Oxidationsstufen +1 oder +2, und Al in der Oxidationsstufe +3 vorkommen. Bevorzugte Metallionen Mⁿ⁺ sind Al³⁺, Ti⁴⁺, Y³⁺, Zr⁴⁺, La³⁺, Ce³⁺, Ce⁴⁺, Sc³⁺, Yb³⁺, Ta⁵⁺ und ihre Mischungen.

Ein Gegenstand der Erfindung ist ein Geschirrspülmittel, insbesondere ein maschinelles Geschirrspülmittel, enthaltend einen Metallkomplex der oben definierten Formel (III) .

Weitere Gegenstände der vorliegenden Erfindung sind die Verwendung eines Metallkomplexes der oben definierten Formel (III) oder eines erfindungsgemäßen Geschirrspülmittels in einem maschinellen Geschirrspülverfahren, insbesondere die Verwendung zur Verbesserung der Reinigungsleistung in einer automatischen Geschirrspülmaschine.

Noch ein weiterer Gegenstand der Erfindung ist ein maschinelles Geschirrspülverfahren, bei dem ein Metallkomplex der oben definierten Formel (III) oder ein erfindungsgemäßes Geschirrspülmittel, insbesondere zu dem Zweck, die Reinigungsleistung zu verbessern, zum Einsatz kommt.

Die oben und im Folgenden für die einzelnen Erfindungsgegenstände beschriebenen besonderen oder bevorzugten Ausführungsformen gelten auch für die anderen Erfindungsgegenstände.

Die erfindungsgemäßen Mittel enthalten, bezogen auf das Gesamtgewicht des Mittels, vorzugsweise 0,001 Gew.-% bis 10 Gew.-%, insbesondere 0,01 Gew.-% bis 3 Gew.-% eines Metallkomplexes der oben definierten Formel (III) und können neben dem erfindungswesentlichen Komplex weitere in derartigen Mitteln üblicherweise enthaltene Bestandteile enthalten, vorzugsweise ausgewählt aus Tensiden, insbesondere nichtionischen Tensiden und/oder anionischen Tensiden, Gerüststoffen, Enzymen, Verdickern, Sequestrierungsmitteln, Elektrolyten, Korrosionsinhibitoren, insbesondere Silberschutzmitteln, Glaskorrosionsinhibitoren, Schauminhibitoren, Farbstoffen, Duftstoffen, Bitterstoffen, antimikrobiellen Wirkstoffen und Desintegrationshilfsmitteln, insbesondere Tensid.

Die Mittel enthalten vorzugsweise mindestens ein nichtionisches Tensid. Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4. Auch nichtionische Tenside vom Typ der Aminoxide, beispielsweise N-Kokosalkyl-N,N-dimethylaminoxid und N-Talgalkyl-N,N-dihy-droxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon. Weitere geeignete Tenside sind die als PHFA bekannten Polyhydroxyfettsäureamide. Bevorzugt werden schwachschäumende nichtionische Tenside eingesetzt, insbesondere alkoxylierte, vor allem ethoxylierte, schwachschäumende nichtionische Tenside. Mit besonderem Vorzug enthalten die maschinellen Geschirrspülmittel nichtionische Tenside aus der Gruppe der alkoxylierten Alkohole. Eine Klasse einsetzbarer nichtionischer Tenside, die entweder als alleiniges nichtionisches Tensid oder in Kombination mit anderen nichtionischen Tensiden eingesetzt werden können, sind demnach alkoxylierte, vorzugsweise ethoxylierte oder ethoxylierte und propoxylierte Fettsäurealkylester, vorzugsweise mit 1 bis 4 Kohlenstoffatomen in der Alkylkette. Bevorzugt einzusetzende Tenside stammen aus den Gruppen der ethoxylierten primären Alkohole und Mischungen dieser Tenside mit strukturell komplizierter aufgebauten Tensiden wie Polyoxypropylen/Polyoxyethylen/Polyoxypropylen ((PO/EO/PO)-Tenside). Solche (PO/EO/PO)-Niotenside zeichnen sich durch gute Schaumkontrolle aus. Niotenside mit alternierenden Ethylenoxid- und Alkylenoxideinheiten können bevorzugt sein. Unter diesen sind wiederum Tenside mit EO-AO-EO-AO-Blöcken bevorzugt, wobei jeweils eine bis zehn EO- oder AO-Gruppen aneinander gebunden sind, bevor ein Block aus den jeweils anderen Gruppen folgt. Hier sind nichtionische Tenside der allgemeinen Formel 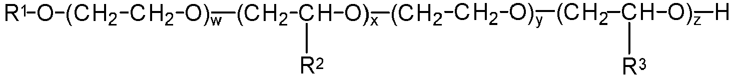 bevorzugt, in der R¹ für einen geradkettigen oder verzweigten, gesättigten oder ein- oder mehrfach ungesättigten C₆₋₂₄-Alkyl- oder -Alkenylrest steht; jede Gruppe R² oder R³ unabhängig voneinander ausgewählt ist aus -CH₃, -CH₂CH₃, -CH₂CH₂-CH₃, CH(CH₃)₂ und die Indizes w, x, y, z unabhängig voneinander für ganze Zahlen von 1 bis 6 stehen. Somit sind insbesondere nichtionische Tenside bevorzugt, die einen C₉₋₁₅-Alkylrest mit 1 bis 4 Ethylenoxideinheiten, gefolgt von 1 bis 4 Propylenoxideinheiten, gefolgt von 1 bis 4 Ethylenoxideinheiten, gefolgt von 1 bis 4 Propylenoxideinheiten aufweisen. Bevorzugte nichtionische Tenside sind hierbei solche der allgemeinen Formel

R¹-CH(OH)CH₂O-(AO)_{w}-(A'O)ₓ-(A"O)_{y}-(A‴O)_{z}-R²,

in der R¹ für einen geradkettigen oder verzweigten, gesättigten oder ein- oder mehrfach ungesättigten C₆₋₂₄-Alkyl- oder -Alkenylrest steht; R² für H oder einen linearen oder verzweigten Kohlenwasserstoffrest mit 2 bis 26 Kohlenstoffatomen steht; A, A', A" und A‴ unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen, und w, x, y und z für Werte zwischen 0,5 und 120 stehen, wobei x, y und/oder z auch 0 sein können. Bevorzugt werden insbesondere solche endgruppenverschlossene, poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht. Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht. Zur Gruppe dieser nichtionischen Tenside zählen beispielsweise die C₂₋₂₆ Fettalkohol-(PO)₁-(EO)₁₅₋₄₀-2-hydroxyalkylether, insbesondere auch die C₈₋₁₀ Fettalkohol-(PO)₁-(EO)₂₂-2-hydroxydecylether. Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- oder mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind. Weitere bevorzugt einsetzbare Niotenside sind die endgruppenverschlossenen poly(oxyal-kylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, isoPropyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x größer oder gleich 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder-CH₂CH₃ besonders bevorzugt. Besonders bevorzugte Werte für x liegen im Bereich von 1 bis 20, insbesondere von 6 bis 15. Dabei kann jedes R³ unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt. Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt. Als besonders wirkungsvoll haben sich schließlich die nichtionischen Tenside der allgemeinen Formel R¹-CH(OH)CH₂O-(AO)_{w}-R² erwiesen, in der R¹ für einen geradkettigen oder verzweigten, gesättigten oder ein- oder mehrfach ungesättigten C₆₋₂₄-Alkyl- oder -Alkenylrest steht; R² für einen linearen oder verzweigten Kohlenwasserstoffrest mit 2 bis 26 Kohlenstoffatomen steht; A für einen Rest aus der Gruppe CH₂CH₂, CH₂CH₂CH₂, CH₂CH(CH₃), vorzugsweise für CH₂CH₂ steht, und w für Werte zwischen 1 und 120, vorzugsweise 10 bis 80, insbesondere 20 bis 40 steht. Zur Gruppe dieser nichtionischen Tenside zählen beispielsweise die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄₋₂₂ Fettalkohol-(EO)₄₀₋₈₀-2-hydroxyalkylether. In verschiedenen Ausführungsformen der Erfindung können anstelle der endgruppenverschlossenen Hydroxymischether auch die entsprechenden nicht endgruppenverschlossenen Hydroxymischether eingesetzt werden. Diese können den obigen Formeln genügen, wobei R² aber Wasserstoff ist und R¹, R³, A, A', A", A‴, w, x, y und z wie oben definiert sind.

Die hierin beschriebenen Mittel, die mindestens ein nichtionisches Tensid, vorzugsweise ein nichtionisches Tensid aus der Gruppe der Hydroxymischether, umfassen, enthalten das Tensid in verschiedenen Ausführungsformen in einer Menge bezogen auf das Gesamtgewicht des Mittels von mindestens 2 Gew.%, vorzugsweise mindestens 5 Gew.%. Die absolut pro Anwendung eingesetzten Mengen können beispielsweise im Bereich von 0,5 g bis 10 g pro Anwendung, vorzugsweise im Bereich von 1 g bis 5 g pro Anwendung liegen.

Als anionische Tenside eignen sich in den Geschirrspülmitteln alle anionischen oberflächenaktiven Stoffe. Diese sind gekennzeichnet durch eine wasserlöslich machende, anionische Gruppe wie zum Beispiel eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Geeignete anionische Tenside liegen vorzugsweise in Form der Natrium-, Kalium- und Ammonium- sowie der Mono-, Di- und Trialkanolammoniumsalze mit 2 bis 4 C-Atomen in der Alkanolgruppe vor. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül. Die Geschirrspülmittel enthalten daher in verschiedenen Ausführungsformen mindestens ein Tensid der Formel R⁴-O-(AO)ₙ-SO₃⁻ X⁺. In dieser Formel steht R⁴ für einen linearen oder verzweigten, substituierten oder unsubstituierten Alkyl-, Aryl- oder Alkylarylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. AO steht für eine Ethylenoxid- (EO) oder Propylenoxid- (PO) Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH4⁺, ½ Zn²⁺,½ Mg²⁺,½ Ca²⁺,½ Mn²⁺, und deren Mischungen. Besonders bevorzugte anionische Tenside werden ausgewählt aus Fettalkoholethersulfaten der Formel A-1 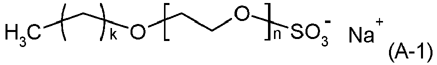 mit k = 11 bis 19, n = 2, 3, 4, 5, 6, 7 oder 8. Ganz besonders bevorzugte Vertreter sind Na-C₁₂₋₁₄ Fettalkoholethersulfate mit 2 EO (k = 11-13, n = 2 in Formel A-1).Die Mittel können ferner zusätzlich oder alternativ mindestens ein Tensid der Formel R⁵-A-SO₃⁻ Y⁺ (A-2) enthalten. In dieser Formel A-2 steht R⁵ für einen linearen oder verzweigten, substituierten oder unsubstituierten Alkyl-, Aryl- oder Alkylarylrest und die Gruppierung -A- für -O- oder eine chemische Bindung. In anderen Worten lassen sich durch die vorstehende Formel Sulfat- (A = O) oder Sulfonat- (A = chemische Bindung) - tenside beschreiben. In Abhängigkeit von der Wahl der Gruppierung A sind bestimmte Reste R⁵ bevorzugt. Bei den Sulfattensiden (A = O) steht R⁵ vorzugsweise für einen linearen, unsubstituierten Alkylrest, besonders bevorzugt für einen Fettalkoholrest. Bevorzugte Reste R⁵ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R⁵ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. Y steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen Y+ können ausgewählt sein aus NH4⁺, ½ Zn²⁺,½ Mg²⁺,½ Ca²⁺,½ Mn²⁺, und deren Mischungen. Solche besonders bevorzugten Tenside sind ausgewählt aus Fettalkoholsulfaten der Formel 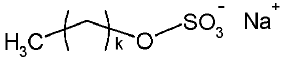 mit k = 11 bis 19. Ganz besonders bevorzugte Vertreter sind Na-C₁₂₋₁₄ Fettalkoholsulfate (k = 11-13). Bei den Sulfonattensiden (A = chemische Bindung in Formel A-2) steht R⁵ vorzugsweise für einen linearen oder verzweigten unsubstituierten Alkylarylrest. Auch hier steht X für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X+ können ausgewählt sein aus NH4⁺, ½ Zn²⁺,½ Mg²⁺,½ Ca²⁺,½ Mn²⁺, und deren Mischungen. Solche Tenside können ausgewählt sein aus linearen oder verzweigten Alkylbenzolsulfonaten.

Anstelle der genannten Tenside oder in Verbindung mit ihnen können auch kationische und/oder amphotere Tenside, wie Betaine oder quartäre Ammoniumverbindungen, eingesetzt werden. Es ist allerdings bevorzugt, dass keine kationischen und/oder amphoteren Tenside eingesetzt werden.

Als Gerüststoffe, die in dem Geschirrspülmittel enthalten sein können, sind insbesondere Silikate, Aluminiumsilikate (insbesondere Zeolithe), Carbonate, organische Di- und Polycarbonsäuren und Aminocarbonsäuren oder deren Salze, und - wo keine ökologischen Vorurteile gegen ihren Einsatz bestehen - auch die Phosphate. Mischungen dieser Stoffe sind ebenfalls einsetzbar.

Es können beispielsweise kristalline schichtförmige Silikate der allgemeinen Formel NaMSiₓO₂ₓ₊₁ · y H₂O eingesetzt werden, worin M Natrium oder Wasserstoff darstellt, x eine Zahl von 1,9 bis 22, vorzugsweise von 1,9 bis 4, wobei besonders bevorzugte Werte für x 2, 3 oder 4 sind, und y für eine Zahl von 0 bis 33, vorzugsweise von 0 bis 20 steht. Die kristallinen schichtförmigen Silikate der Formel NaMSiₓO₂ₓ₊₁ · y H₂O werden beispielsweise von der Firma Clariant GmbH (Deutschland) unter dem Handelsnamen Na-SKS vertrieben. Beispiele für diese Silikate sind Na-SKS-1 (Na₂Si₂₂O₄₅ · x H₂O, Kenyait), Na-SKS-2 (Na₂Si₁₄O₂₉ · x H₂O, Magadiit), Na-SKS-3 (Na₂Si₈O₁₇ · x H₂O) oder Na-SKS-4 (Na₂Si₄O₉ · x H₂O, Makatit). Für die Zwecke der vorliegenden Erfindung besonders geeignet sind kristalline Schichtsilikate der Formel NaMSiₓO₂ₓ₊₁ · y H₂O, in denen x für 2 steht. Insbesondere sind sowohl ß- als auch δ-Natriumdisilikate Na₂Si₂O₅ · y H₂O sowie weiterhin vor allem Na-SKS-5 (α-Na₂Si₂O₅), Na-SKS-7 (ß-Na₂Si₂O₅, Natrosilit), Na-SKS-9 (NaHSi₂O₅ · H₂O), Na-SKS-10 (NaHSi₂O₅ · 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅) bevorzugt. Maschinelle Geschirrspülmittel können gewünschtenfalls einen Gewichtsanteil des kristallinen schichtförmigen Silikats der Formel NaMSiₓO₂ₓ₊₁ · y H₂O von 0,1 bis 20 Gew.-%, bevorzugt von 0,2 bis 15 Gew.-% und insbesondere von 0,4 bis 10 Gew.-% enthalten, jeweils bezogen auf das Gesamtgewicht dieser Mittel.

Einsetzbar sind auch amorphe Natriumsilikate mit einem Modul Na₂O:SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen. Die Löseverzögerung gegenüber herkömmlichen amorphen Natriumsilikaten kann dabei beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" verstanden, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen, hervorrufen.

Im Rahmen der vorliegenden Erfindung ist es bevorzugt, dass diese(s) Silikat(e), vorzugsweise Alkalisilikate, besonders bevorzugt kristalline oder amorphe Alkalidisilikate, in den Mitteln in Mengen von 1 bis 40 Gew.-%, vorzugsweise von 2 bis 35 Gew.-% jeweils bezogen auf das Gewicht des maschinellen Geschirrspülmittels, enthalten sind.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- oder Pentakaliumtriphosphat (Natrium- oder Kaliumtripolyphosphat) in der Wasch- oder Geschirrspülmittel-Industrie die größte Bedeutung. Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen und Kalkinkrustationen in Geweben und tragen überdies zur Reinigungsleistung bei. Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat) und entsprechende Mischsalze (Natriumkaliumtripolyphosphate). Vorzugsweise sind die Mittel aber phosphatfrei. Werden im Rahmen der vorliegenden Anmeldung Phosphate als reinigungsaktive Substanzen im maschinellen Geschirrspülmittel eingesetzt, so enthalten bevorzugte Mittel diese(s) Phosphat(e), vorzugsweise Alkalimetallphosphat(e), besonders bevorzugt Pentanatrium- oder Pentakaliumtriphosphat (Natrium- oder Kaliumtripolyphosphat), in Mengen von 5 bis 80 Gew.-%, vorzugsweise von 10 bis 60 Gew.-% und insbesondere von 18 bis 45 Gew.-%, jeweils bezogen auf das Gewicht des maschinellen Geschirrspülmittels.

Die Geschirrspülmittel können als weiteren Gerüststoff insbesondere auch Phosphonate enthalten. Als Phosphonat-Verbindung wird vorzugsweise ein Hydroxyalkan- und/oder Aminoalkanphosphonat eingesetzt. Unter den Hydroxyalkanphosphonaten ist das 1-Hydroxyethan-1,1-diphosphonat (HEDP) von besonderer Bedeutung. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Phosphonate sind in den Mitteln vorzugsweise in Mengen von 0,1 bis 10 Gew.-%, insbesondere in Mengen von 0,5 bis 8 Gew.-%, jeweils bezogen auf das Gesamtgewicht des Geschirrspülmittels, enthalten.

Weitere Gerüststoffe sind die Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden können. Besonders bevorzugt ist ein Buildersystem enthaltend eine Mischung aus Tripolyphosphat und Natriumcarbonat. Ebenfalls besonders bevorzugt ist ein Buildersystem enthaltend eine Mischung aus Tripolyphosphat und Natriumcarbonat und Natriumdisilikat. Aufgrund ihrer im Vergleich mit anderen Buildersubstanzen geringen chemischen Kompatibilität mit den übrigen Inhaltsstoffen von maschinellen Geschirrspülmitteln, werden die optionalen Alkalimetallhydroxide bevorzugt nur in geringen Mengen, vorzugsweise in Mengen unterhalb 10 Gew.-%, bevorzugt unterhalb 6 Gew.-%, besonders bevorzugt unterhalb 4 Gew.-% und insbesondere unterhalb 2 Gew.-%, jeweils bezogen auf das Gesamtgewicht des maschinellen Geschirrspülmittels, eingesetzt. Besonders bevorzugt werden Mittel, welche bezogen auf ihr Gesamtgewicht weniger als 0,5 Gew.-% und insbesondere keine Alkalimetallhydroxide enthalten. Besonders bevorzugt ist der Einsatz von Carbonat(en) und/oder Hydrogencarbonat(en), vorzugsweise Alkalicarbonat(en), besonders bevorzugt Natriumcarbonat, in Mengen von 2 bis 50 Gew.-%, vorzugsweise von 5 bis 40 Gew.-% und insbesondere von 7,5 bis 30 Gew.-%, jeweils bezogen auf das Gewicht des maschinellen Geschirrspülmittels. Besonders bevorzugt werden Mittel, welche bezogen auf das Gewicht des maschinellen Geschirrspülmittels weniger als 20 Gew.-%, vorzugsweise weniger als 17 Gew.-%, bevorzugt weniger als 13 Gew.-% und insbesondere weniger als 9 Gew.-% Carbonat(e) und/oder Hydrogencarbonat(e), vorzugsweise Alkalicarbonat(e), besonders bevorzugt Natriumcarbonat enthalten.

Als organische Gerüststoffe sind insbesondere Polycarboxylate / Polycarbonsäuren, polymere Polycarboxylate, Asparaginsäure, Polyacetale, Dextrine, weitere organische Cobuilder sowie die bereits oben als Gerüststoffe genannten Phosphonate zu nennen. Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes der maschinellen Geschirrspülmittel. Insbesondere sind hierbei Citronensäure, Bernsteinsäure, Glutarsäure, Adipinsäure, Gluconsäure und beliebige Mischungen aus diesen zu nennen. Als besonders vorteilhaft für die Reinigungs- und Klarspülleistung der Mittel hat sich der Einsatz von Citronensäure und/oder Citraten erwiesen. Bevorzugt sind daher maschinelle Geschirrspülmittel, die Citronensäure oder ein Salz der Citronensäure enthalten. Eine weitere bedeutende Klasse der phosphatfreien Gerüststoffe stellen Aminocarbonsäuren und/oder ihre Salze dar. Besonders bevorzugte Vertreter dieser Klasse sind Methylglycindiessigsäure (MGDA) oder ihre Salze sowie Glutamindiessigsäure (GLDA) oder ihre Salze oder Ethylendiamindiessigsäure oder ihre Salze (EDDS). Der Gehalt an diesen Aminocarbonsäuren oder ihren Salzen kann beispielsweise zwischen 0,1 und 30 Gew.-% bevorzugt zwischen 1 und 25 Gew.-% und insbesondere zwischen 5 und 20 Gew.-% ausmachen. Aminocarbonsäuren und ihre Salze können zusammen mit den vorgenannten Gerüststoffen, auch mit den phosphatfreien Gerüststoffen, eingesetzt werden.

Die Geschirrspülmittel können ferner ein Sulfopolymer enthalten. Der Gewichtsanteil des Sulfopolymers am Gesamtgewicht des Geschirrspülmittels beträgt vorzugsweise von 0,1 bis 20 Gew.-%, insbesondere von 0,5 bis 18 Gew.-%, besonders bevorzugt 1,0 bis 15 Gew.-%, insbesondere von 4 bis 14 Gew.-%, vor allem von 6 bis 12 Gew.-%. Das Sulfopolymer wird üblicherweise in Form einer wässrigen Lösung eingesetzt, wobei die wässrigen Lösungen typischerweise 20 bis 70 Gew.-%, insbesondere 30 bis 50 Gew.-%, vorzugsweise etwa 35 bis 40 Gew.-% Sulfopolymere enthalten. Als Sulfopolymer wird vorzugsweise ein copolymeres Polysulfonat, vorzugsweise ein hydrophob modifiziertes copolymeres Polysulfonat, eingesetzt. Die Copolymere können zwei, drei, vier oder mehr unterschiedliche Monomereinheiten aufweisen. Bevorzugte copolymere Polysulfonate enthalten neben Sulfonsäuregruppen-haltigem(n) Monomer(en) wenigstens ein Monomer aus der Gruppe der ungesättigten Carbonsäuren. Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist. Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren. Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, OH oder-COOH substituierte Alkyl- oderAlkenylreste oderfür-COOH oder-COOR⁴ steht, wobei R⁴ ein gesättigter oder ungesättigter, geradkettiger oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, COO-(CH₂)ₖ- mit k = 1 bis 6, C(O)-NH-C(CH₃)₂-, C(O)-NH-C(CH₃)₂-CH₂- und -C(O)-NH-CH(CH₃) CH₂-. Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃ und - CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, C(O)-NH-C(CH₃)₂-, C(O)-NH-C(CH₃)₂-CH₂- und -C(O)-NH-CH(CH₃) CH₂-. Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze. In den Polymeren können die Sulfonsäuregruppen ganz oder teilweise in neutralisierter Form vorliegen, das heißt dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten sulfonsäuregruppenhaltigen Copolymeren ist erfindungsgemäß bevorzugt. Die Monomerenverteilung der bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.- %, die Monomere sind hierbei vorzugsweise ausgewählt aus den zuvor genannten. Die Molmasse der bevorzugt eingesetzten Sulfo-Copolymere kann variiert werden, um die Eigenschaften der Polymere dem gewünschten Verwendungszweck anzupassen. Bevorzugte Geschirrspülmittel sind dadurch gekennzeichnet, dass die Copolymere Molmassen von 2000 g/mol bis 200 000 g/mol, vorzugsweise von 4000 g/mol bis 25 000 g/mol und insbesondere von 5000 g/mol bis 15 000 g/mol aufweisen.

Die Geschirrspülmittel können ferner weitere Polymere enthalten. Zur Gruppe geeigneter Polymere zählen insbesondere die reinigungsaktiven Polymere, beispielsweise die Klarspülpolymere und/oder als Enthärter wirksame Polymere. Bevorzugte einsetzbare Polymere stammen aus der Gruppe der Alkylacrylamid/Acrylsäure-Copolymere, der Alkylacrylamid/Methacrylsäure-Copolymere, der Alkylacrylamid/Methylmethacrylsäure-Copolymere, der Alkylacrylamid/Acrylsäure/Alkyl-aminoalkyl(meth)acrylsäure-Copolymere, der Alkylacrylamid/Methacrylsäure/Alkylaminoalkyl(meth)-acrylsäure-Copolymere, der Alkylacrylamid/Methylmethacrylsäure/Alkylaminoalkyl(meth)acrylsäure-Copolymere, der Alkylacrylamid/Alkymethacrylat/Alkylaminoethylmethacrylat/Alkylmethacrylat-Copolymere sowie der Copolymere aus ungesättigten Carbonsäuren, kationisch derivatisierten ungesättigten Carbonsäuren und gegebenenfalls weiteren ionischen oder nichtionogenen Monomeren. Weitere einsetzbare Polymere stammen aus der Gruppe der Acrylamidoalkyltrialkylammoniumchlorid/Acrylsäure-Copolymere sowie deren Alkali- und Ammoniumsalze, der Acrylamidoalkyltrialkylammoniumchlorid/Methacrylsäure-Copolymere sowie deren Alkali- und Ammoniumsalze und der Methacroylethylbetain/Methacrylat-Copolymere. Einsetzbare kationische Polymere stammen aus den Gruppen der quaternierten Cellulose-Derivate, der Polysiloxane mit quaternären Gruppen, der kationischen Guar-Derivate, der polymeren Dimethyldiallylammoniumsalze und deren Copolymere mit Acrylsäure und Methacrylsäure und deren Estern und Amiden, der Copolymere des Vinylpyrrolidons mit quaternierten Derivaten des Dialkylaminoacrylats und -methacrylats, der Vinylpyrrolidon-Methoimidazoliniumchlorid-Copolymere, der quaternierter Polyvinylalkohole oder der unter den INCI-Bezeichnungen Polyquaternium 2, Polyquaternium 17, Polyquaternium 18 und Polyquaternium 27 angegeben Polymere.

Vorzugsweise enthalten die Mittel der vorliegenden Erfindung mindestens eine Enzymzubereitung oder Enzymzusammensetzung, die ein oder mehrere Enzyme enthalten. Geeignete Enzyme umfassen, ohne darauf beschränkt zu sein, Proteasen, Amylasen, Lipasen, Hemicellulasen, Cellulasen, Perhydrolasen oder Oxidoreduktasen, sowie vorzugsweise deren Gemische. Diese Enzyme sind im Prinzip natürlichen Ursprungs; ausgehend von den natürlichen Molekülen stehen für den Einsatz in Geschirrspülmitteln verbesserte Varianten zur Verfügung, die entsprechend bevorzugt eingesetzt werden. Die Mittel enthalten Enzyme vorzugsweise in Gesamtmengen von 1 × 10⁻⁶ bis 5 Gew.-% bezogen auf aktives Protein. Die Proteinkonzentration kann mit Hilfe bekannter Methoden, zum Beispiel dem BCA-Verfahren oder dem Biuret-Verfahren bestimmt werden.

Proteasen gehören zu den technisch bedeutendsten Enzymen überhaupt. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen. Beispiele für die in Wasch- und Geschirrspülmitteln bevorzugt eingesetzten Proteasen vom Subtilisin-Typ sind die Subtilisine BPN' und Carlsberg, die Protease PB92, die Subtilisine 147 und 309, die Protease aus Bacillus lentus, insbesondere aus Bacillus lentus DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Geschirrspülmitteln optimiert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

Beispiele für einsetzbare Amylasen sind die α-Amylasen aus Bacillus licheniformis, aus B. amyloliquefaciens, aus B. stearothermophilus, aus Aspergillus niger und A. oryzae sowie die für den Einsatz in Geschirrspülmitteln verbesserten Weiterentwicklungen der vorgenannten Amylasen. Des Weiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben.

Einsetzbar sind weiterhin Lipasen oder Cutinasen, insbesondere wegen ihrer Triglycerid-spaltenden Aktivitäten, aber auch, um aus geeigneten Vorstufen in situ Persäuren zu erzeugen. Hierzu gehören beispielsweise die ursprünglich aus Humicola lanuginosa (Thermomyces lanuginosus) erhältlichen, beziehungsweise weiterentwickelten Lipasen, insbesondere solche mit dem Aminosäureaustausch D96L.

Weiterhin können Enzyme eingesetzt werden, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen.

Zur Erhöhung der bleichenden Wirkung können Oxidoreduktasen, beispielsweise Oxidasen, Oxygenasen, Katalasen, Peroxidasen, wie Halo-, Chloro-, Bromo-, Lignin-, Glucose- oder Mangan-Peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Ein Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Geschirrspülmittel können zu diesem Zweck Stabilisatoren enthalten; die Bereitstellung derartiger Mittel stellt eine bevorzugte Ausführungsform der vorliegenden Erfindung dar.

Reinigungsaktive Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Das Enzym-Protein bildet in aller Regel nur einen Bruchteil des Gesamtgewichts üblicher Enzym-Zubereitungen. Bevorzugt eingesetzte Enzym-Zubereitungen enthalten zwischen 0,1 und 40 Gew.- %, bevorzugt zwischen 0,2 und 30 Gew.-%, besonders bevorzugt zwischen 0,4 und 20 Gew.-% und insbesondere zwischen 0,8 und 10 Gew.-% des Enzymproteins.

Bevorzugt werden insbesondere solche Geschirrspülmittel, die, jeweils bezogen auf ihr Gesamtgewicht, 0,1 bis 12 Gew.-%, vorzugsweise 0,2 bis 10 Gew.-% und insbesondere 0,5 bis 8 Gew.-% Enzym-Zubereitungen enthalten.

Die hierin beschriebenen Zusammensetzungen können auch Enzymstabilisatoren beinhalten. Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet. Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Weitere Enzymstabilisatoren sind dem Fachmann aus dem Stand der Technik bekannt.

Bleichmittel sind reinigungsaktive Substanzen. Unterden als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Einsetzbar sind außerdem alle weiteren dem Fachmann aus dem Stand der Technik bekannten anorganischen oder organischen Peroxybleichmittel. Als Bleichmittel werden die Percarbonate und hier insbesondere Natriumpercarbonat besonders bevorzugt. Die Geschirrspülmittel können, in verschiedenen Ausführungsformen, 1 Gew.-% bis 35 Gew.-%, vorzugsweise 2,5 Gew.-% bis 30 Gew.-%, besonders bevorzugt 3,5 Gew.-% bis 20 Gew.-% und insbesondere 5 Gew.-% bis 15 Gew.-% Bleichmittel, vorzugsweise Natriumpercarbonat, enthalten.

In verschiedenen Ausführungsformen der Erfindung enthalten die maschinellen Geschirrspülmittel zusätzlich mindestens einen Bleichaktivator. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Von allen dem Fachmann aus dem Stand der Technik bekannten Bleichaktivatoren werden mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- oder iso-NOBS) besonders bevorzugt eingesetzt. Auch Kombinationen konventioneller Bleichaktivatoren können eingesetzt werden. Als Bleichaktivator wird TAED, insbesondere in Kombination mit einem Percarbonat-Bleichmittel, vorzugsweise Natriumpercarbonat, ganz besonders bevorzugt. Diese Bleichaktivatoren werden vorzugsweise in Mengen bis 10 Gew.-%, insbesondere 0,1 Gew.-% bis 8 Gew.-%, besonders 2 Gew.-% bis 8 Gew.-% und besonders bevorzugt 2 Gew.-% bis 6 Gew.-%, jeweils bezogen auf das Gesamtgewicht der Mittel, eingesetzt.

Generell kann der pH-Wert des Geschirrspülmittels mittels üblicher pH-Regulatoren eingestellt werden, wobei der pH-Wert abhängig von dem gewünschten Einsatzzweck gewählt wird. In verschiedenen Ausführungsformen liegt der pH-Wert in einem Bereich von 5,5 bis 10,5, vorzugsweise 5,5 bis 9,5, noch bevorzugter 7 bis 9, insbesondere größer 7, vor allem im Bereich 7,5 bis 8,5. Als pH-Stellmittel dienen Säuren und/oder Alkalien, vorzugsweise Alkalien. Geeignete Säuren sind insbesondere organische Säuren wie die Essigsäure, Zitronensäure, Glycolsäure, Milchsäure, Bernsteinsäure, Adipinsäure, Äpfelsäure, Weinsäure und Gluconsäure oder auch Amidosulfonsäure. Daneben können aber auch die Mineralsäuren Salzsäure, Schwefelsäure und Salpetersäure oder deren Mischungen eingesetzt werden. Geeignete Basen stammen aus der Gruppe der Alkali- und Erdalkalimetallhydroxide und -carbonate, insbesondere der Alkalimetallhydroxide, von denen Kaliumhydroxid und vor allem Natriumhydroxid bevorzugt ist. Besonders bevorzugt ist allerdings flüchtiges Alkali, beispielsweise in Form von Ammoniak und/oder Alkanolaminen, die bis zu 9 C-Atome im Molekül enthalten können. Das Alkanolamin ist hierbei vorzugsweise ausgewählt aus der Gruppe bestehend aus Mono-, Di-, Triethanol- und -Propanolamin und deren Mischungen. Zur Einstellung und/oder Stabilisierung des pH-Werts kann das erfindungsgemäße Mittel auch ein oder mehrere Puffersubstanzen (INCI Buffering Agents) enthalten, üblicherweise in Mengen von 0,001 bis 5 Gew.-%. Bevorzugt sind Puffersubstanzen, die zugleich Komplexbildner oder sogar Chelatbildner (Chelatoren, INCI Chelating Agents) sind. Besonders bevorzugte Puffersubstanzen sind Citronensäure und die Citrate, insbesondere die Natrium- und Kaliumcitrate, beispielsweise Trinatriumcitrat·2H₂O und Trikaliumcitrat·H₂O.

Glaskorrosionsinhibitoren verhindern das Auftreten von Trübungen, Schlieren und Kratzern, aber auch das Irisieren der Glasoberfläche von maschinell gereinigten Gläsern. Bevorzugte Glaskorrosionsinhibitoren stammen aus der Gruppe der Magnesium- und Zinksalze sowie der Magnesium- und Zinkkomplexe. Im Rahmen der vorliegenden Erfindung liegt der Gehalt an Zinksalz in Geschirrspülmitteln vorzugsweise im Bereich von 0,1 Gew.-% bis 5 Gew.-%, bevorzugt von 0,2 Gew.-% bis 4 Gew.-% und insbesondere von 0,4 Gew.-% bis 3 Gew.-%, jeweils bezogen auf das Gesamtgewicht des Glaskorrosionsinhibitor-haltigen Mittels.

Als Parfümöle oder Duftstoffe können im Rahmen der vorliegenden Erfindung einzelne Riechstoffverbindungen, zum Beispiel die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pinien-, Citrus-, Jasmin-, Patchouli-, Rosen- oder Ylang-Ylang-Öl.

Weiterhin können Konservierungsmittel in den Mitteln enthalten sein. Geeignet sind beispielsweise Konservierungsmittel aus den Gruppen der Alkohole, Aldehyde, antimikrobiellen Säuren und/oder deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, Iod, Iodophore und Peroxide. Bevorzugte antimikrobielle Wirkstoffe werden vorzugsweise ausgewählt aus der Gruppe umfassend Ethanol, n-Propanol, i-Propanol, 1,3-Butandiol, Phenoxyethanol, 1,2-Propylenglykol, Glycerin, Undecylensäure, Zitronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octan-amin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine. Besonders bevorzugte Konservierungsmittel sind jedoch ausgewählt aus der Gruppe umfassend Salicylsäure, quaternäre Tenside, insbesondere Benzalkoniumchlorid und Isothiazole und deren Derivate wie Isothiazoline und Isothiazolinone.

Generell kann die Konfektionierung hierin beschriebener maschineller Geschirrspülmittel in unterschiedlicher Weise erfolgen. Die Mittel können in fester oder flüssiger sowie als Kombination fester und flüssiger Angebotsformen vorliegen. Als feste Angebotsformen eignen sich insbesondere Pulver, Granulate, Extrudate, Kompaktate, insbesondere Tabletten. Die flüssigen Angebotsformen auf Basis von Wasser und/oder organischen Lösungsmitteln können verdickt, in Form von Gelen vorliegen. Die Mittel können in Form einphasiger oder mehrphasiger Produkte konfektioniert werden. Die einzelnen Phasen mehrphasiger Mittel können gleiche oder unterschiedliche Aggregatzustände aufweisen.

Die Geschirrspülmittel können als Formkörper vorliegen. Um den Zerfall solcher vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln oder Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.-%, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Die hierin beschriebenen maschinellen Geschirrspülmittel werden vorzugsweise zu Dosiereinheiten vorkonfektioniert. Diese Dosiereinheiten umfassen vorzugsweise die für einen Reinigungsgang notwendige Menge an reinigungsaktiven Substanzen. Bevorzugte Dosiereinheiten weisen ein Gewicht zwischen 12 und 30 g, bevorzugt zwischen 14 und 26 g und insbesondere zwischen 16 und 22 g auf. Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml.

Die maschinellen Geschirrspülmittel, insbesondere die vorgefertigten Dosiereinheiten weisen mit besonderem Vorzug eine wasserlösliche Umhüllung auf. Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Die wasserlösliche Verpackung kann eine oder mehr Kammern aufweisen. Das Mittel kann in einer oder mehreren Kammern, falls vorhanden, der wasserlöslichen Umhüllung enthalten sein. Die Menge an Mittel entspricht vorzugsweise der vollen oder halben Dosis, die für einen Spülgang benötigt wird.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf. Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10 000 g/mol bis 1 000 000 g/mol, vorzugsweise von 20 000 g/mol bis 500000 g/mol, besonders bevorzugt von 30 000 g/mol bis 100000 g/mol und insbesondere von 40 000 g/mol bis 80 000 g/mol liegt. Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 Mol-% bis 100 Mol-%, vorzugsweise 80 Mol-% bis 90 Mol-%, besonders bevorzugt 81 Mol-% bis 89 Mol-% und insbesondere 82 Mol-% bis 88 Mol-% ausmacht. Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren. Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist. Ebenfalls bevorzugte Polyvinylalkoholcopolymere umfassen neben Vinylalkohol eine ethylenisch ungesättigte Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus. Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus. Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon^{®} PT, Solub-Ion^{®} GA, Solublon^{®} KC oder Solublon^{®} KL von der Aicello Chemical Europe GmbH oder die Folien VF-HP von Kuraray.

Beim erfindungsgemäßen maschinellen Geschirrspülverfahren wird das erfindungsgemäße Mittel während des Durchlaufens eines Geschirrspülprogramms vor Beginn des Hauptspülgangs oder im Verlauf des Hauptspülgangs in den Innenraum einer Geschirrspülmaschine eindosiert. Die Eindosierung oder der Eintrag des erfindungsgemäßen Mittels in den Innenraum der Geschirrspülmaschine kann manuell erfolgen, vorzugsweise wird das Mittel jedoch mittels der Dosierkammer in den Innenraum der Geschirrspülmaschine dosiert.

### Beispiele

### Beispiel 1: Ligandenherstellung

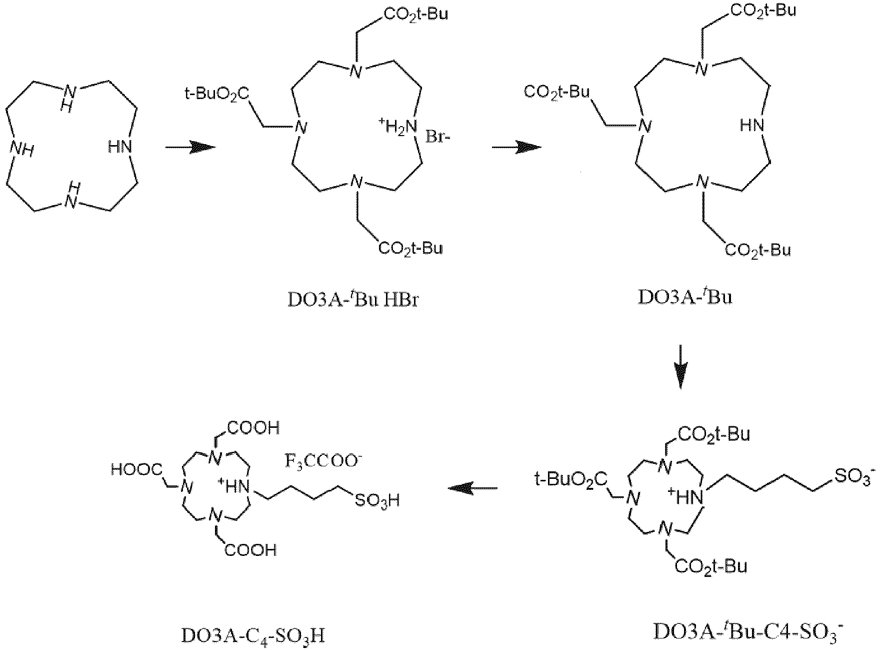

Die beiden ersten Schritte sind veröffentlicht von B. Jagadish et al. in Tetrahedron Letters 52 (2011) 2058-2061.

Zu einer kräftig gerührten Mischung von 5,00 g Tetraazacyclodecan (29 mmol) und 7,87 g Natriumacetat in 60 ml N,N-Dimethylacetamid wurde bei -20 °C innerhalb von 30 Minuten eine Lösung von 18,7 g Bromessigsäure-*tert*-butylester (96 mmol) in 20 ml N,N-Dimethylacetamid zugetropft. Anschließend wurde die Reaktionsmischung 24 Stunden bei Raumtemperatur gerührt, danach zu 300 ml destilliertem Wasser gegeben und 15 g KHCO₃ wurden zugegeben. Der sich bildende Niederschlag wurde abfiltriert und in 250 ml Chloroform gelöst. Nach Waschen mit 100 ml Wasser und Trocknen mit Magnesiumsulfat wurde die Lösung auf ein Volumen von etwa 25 ml eingeengt, 250 ml Diethylether wurden zugegeben und der sich bildende Niederschlag wurde abfiltriert, zweimal mit Diethylether gewaschen und im Vakuum getrocknet. Man erhielt 12,37 g (71 %) DO3A-^{t}Bu HBr, das mittels ¹H-NMR Spektroskopie in CDCl₃ und ESI-MS charakterisiert wurde.

Zu einer Lösung von 5,00 g des so erhaltenen Hydrobromids (8,4 mmol) in 250 ml destilliertem Wasser wurden bei 40 °C 9,4 ml einer 10-gewichtsprozentigen wässrigen KOH-Lösung zugegeben, die Reaktionsmischung wurde 30 Minuten lang gerührt und anschließend 3 mal mit je 100 ml Hexan extrahiert. Die vereinigten organischen Phasen wurden 3 mal mit je 100 ml destilliertem Wasser gewaschen und mit Magnesiumsulfat getrocknet. Nach Entfernen des Lösungsmittels unter vermindertem Druck ergab sich ein farbloses Öl, das sich bei Abkühlung auf -20°C verfestigte. Man erhielt 3,80 g (88 %) DO3A-^{t}Bu, das mittels ¹H-NMR Spektroskopie in CDCl₃ und ESI-MS charakterisiert wurde.

Zu einer zum Sieden unter Rückfluss erhitzten Lösung von 1,60 g der so erhaltenen freien Base (3,1 mmol) in 20 ml wasserfreiem Tetrahydrofuran wurde unter N₂-Atmosphäre eine Lösung von 0,45 g frisch destilliertem 1,4,-Butansulton (3,3 mmol) innerhalb von 20 Minuten zugegeben. Nach Erhitzen zum Sieden unter Rückfluss für 48 Stunden wurde das Lösungsmittel im Vakuum abgezogen und der Rückstand wurde in Methanol/Diethylether (Volumenverhältnis 1:30) aufgenommen, auf 40 °C erwärmt und anschließend über Nacht bei -20°C gehalten. Der ausgefallene Niederschlag wurde mit Hilfe einer Glasfritte abfiltriert, 3 Mal mit je 20 ml Diethylether gewaschen und im Vakuum getrocknet. Man erhielt 1,53 g (76 %) DO3A-*^{t}*Bu-C4-SO3⁻, das mittels ¹H-, ¹³C-, ¹⁵N-NMR und ATR-IR Spektroskopie und ESI-MS charakterisiert wurde.

1,53 g des so erhaltenen zwitterionischen Esters wurden unter N₂-Atmosphäre in 20 ml Trifluoressigsäure bei 25 °C 24 Stunden lang kräftig gerührt. Anschließend wurde Trifluoressigsäure im Vakuum entfernt, der farblose Rückstand wurde in 2 ml Methanol aufgenommen und bei 5 °C mit 50 ml Diethylether versetzt. Nach 2 stündigem Rühren bei 25 °C wurde der ausgefallene Niederschlag mit Hilfe einer Glasfritte abfiltriert und im Vakuum getrocknet. Man erhielt in quantitativer Ausbeute das DO3A-*^{t}*Bu-C4-SO₃H Trifluoracetat, das mittels ¹H-, ¹³C-NMR und ATR-IR Spektroskopie und ESI-MS charakterisiert wurde.

### Beispiel 2: Weitere Ligandenherstellung

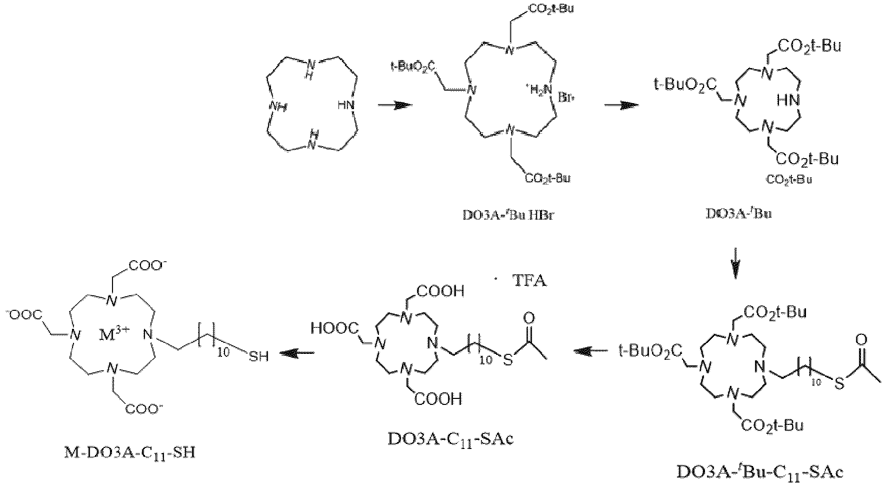

Zu einer zum Sieden unter Rückfluss erhitzten Lösung von 0,5 g der als Zwischenprodukt in Beispiel 1 hergestellten freien Base DO3A-^{t}Bu (1 mmol) und 0,12 g Triethylamin (1,2 mmol) in 20 ml wasserfreiem CHCl₃ wurde unter N₂-Atmosphäre eine Lösung von 0,33 g S-(11-Bromundeceyl)-thioacetat (1,07 mmol) in10 ml CHCl₃ tropfenweise zugegeben. Nach Erhitzen zum Sieden unter Rückfluss für 24 Stunden wurde das Reaktionsgemisch auf Raumtemperatur abgekühlt und mit 20 ml destilliertem Wasser gewaschen. Nach Trocknen mit MgSO₄ wurde das Lösungsmittel im Vakuum abgezogen. Man erhielt in quantitativer Ausbeute DO3A-*^{t}*Bu-C11-SAc als schwach gelben Feststoff.

¹H-NMR (400 MHz, CDCl₃): δ 1.23 - 1.37 (br m, 18 H), 1.43 - 1.47 (br s, 27 H), 2.31 (s, 3 H), 2.50 - 3.23 (br m, 18 H), 3.26 - 3.46 (br m, 8 H).

ESI-MS (+): 743.530 ([M+H]+, berechnet 743.535).

Eine Lösung von 0,63 g des so erhaltenen Esters in 10 ml CH₂Cl₂ wurde unter N₂-Atmosphäre mit 20 ml Trifluoressigsäure versetzt und bei 25 °C 48 Stunden lang gerührt. Anschließend wurden die Lösungsmittel unter reduziertem Druck entfernt, der gelbe ölige Rückstand wurde in 2 ml Methanol aufgenommen und bei 5 °C mit 50 ml Diethylether versetzt. Nach 4 stündigem Rühren bei 25 °C wurde der ausgefallene Niederschlag durch Dekantieren, mehrmaliges Waschen mit Diethylether und Trocknen im Vakuum isoliert. Man erhielt in 82 %-iger Ausbeute das DO3A-C11-SAc Trifluoracetat.

¹H-NMR (400 MHz, D₂O): δ 1.25 - 1.45 (br m, 14 H), 1.52 - 1.66 (q, 2 H), 1.68 - 1.85 (m, 2 H), 2.35 - 2.43 (s, 3 H), 2.87 - 2.95 (t, 2 H), 3.03 - 3.31 (m, 12 H), 3.39 - 3.67 (m, 10 H), 3.91 (s, 2 H).

ESI-MS (+): 575.380 ([M+H]+, berechnet 575.347).

Zur Entfernung der Acetat-Schutzgruppe wurde eine Lösung von 0,34 g des so erhaltenen Trifluoressigsäure-Addukts in 10 ml 7 M NH₃ in wasserfreiem CH₃OH für 15 Minuten unter N₂-Atmosphäre gerührt.

### Beispiel 3: Synthese von Metallkomplexen

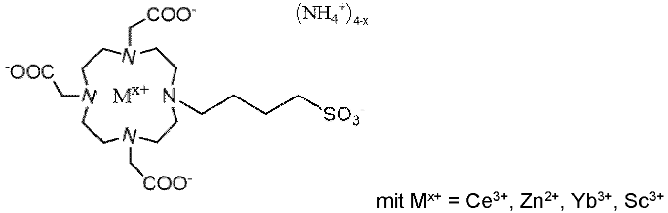

Zu Lösungen von 597 mg des in Beispiel 1 erhaltenen Trifluoracetats in 10 ml 7 M NH₃ in wasserfreiem Methanol unter N₂-Atmosphäre wurden Lösungen von 246 mg CeCl₃, 136 mg ZnCl₂, 279 mg YbCl₃ oder 151 mg ScCl₃ in 5 ml trockenem Methanol zugetropft. Die Reaktionsmischungen wurden 48 Stunden lang unter Rückfluss zum Sieden erhitzt, anschließend auf Raumtemperatur abgekühlt und das Lösungsmittel wurde im Vakuum entfernt. Der Rückstand wurde in 20 ml zweifach destilliertem Wasser aufgenommen, 5 Minuten mit Ultraschall behandelt und Ungelöstes wurde abfiltriert. Nach Entfernen des Lösungsmittels erhielt man in quantitativer Ausbeute die Ce-, Zn-, Yb- und Sc-Komplexe als farblose Feststoffe.

### Beispiel 4: Synthese eines weiteren Metallkomplexes

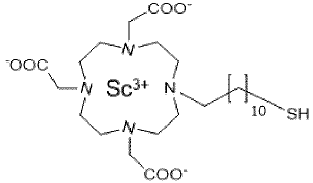

Zu der in Beispiel 2 hergestellten Lösung wurde unter N₂-Atmosphäre eine Lösung von 78 mg wasserfreiem ScCl₃ (0,52 mmol) in 5 ml trockenem Methanol tropfenweise zugegeben. Die Reaktionsmischung wurde 72 Stunden lang auf 75 °C erhitzt und nach Entfernen ungelöster Rückstände mit Hilfe eines Spritzenfilters wurde das Lösungsmittel im Vakuum entfernt. Der feste Rückstand wurde aus CH₃OH/Diethylether (1:50) umkristallisiert; man erhielt nach Trocknen 0,29 g des Sc-Komplexes als farblosen Feststoff, der mittels ESI-MS charakterisiert wurde.

¹H-NMR (600 MHz, MeOH-d4): δ 1,20 -1,70 (m, 18 H), 2,42 -3,26 (m, 18 H), 3,38 - 4.00 (m, 8H). ¹³C-NMR (125 MHz, MeOH-d4): δ 18,9, 19,0, 22,8, 26,6, 26,9, 27,2, 28,0, 28,5, 28,6, 50,6, 53,5, 54,2, 55,3, 64,6, 65,1, 65,3, 65,6, 65,9, 66,2, 174,2, 177,6.

### Beispiel 5: Hydrolyse von Proteinanschmutzung

Die Hydrolyse von Proteinschmutz wurde anhand des Abbaus von Ovalbumin (Hühnerei-Albumin, etwa 45 kDa) untersucht. Dazu wurde eine wässrige Ovalbumin-Lösung (Konzentration an Ovalbumin 0,02 mM) mit einem unten angegebenen Komplex (Konzentration des Komplexes 1 mM) versetzt, so dass die Konzentration an Ovalbumin 0,02 mM und die Konzentration des Komplexes 1 mM betrug, und bei 60°C und pH 6 (eingestellt mit NaOH und HCl) für 1 Stunde, 5 Stunden oder 24 Stunden inkubiert. Anschließend wurde das inkubierte Material mit der bekannten Methode der SDS-PAGE bezüglich der Intensität der dem Ovalbumin zuzuordnenden Bande untersucht. Als Referenz diente unter den gleichen Bedingungen inkubiertes Ovalbumin ohne Komplexzusatz. Die in der nachfolgenden Tabelle 1 angegebenen Intensitätswerte sind relative Werte bezogen auf die Intensität der Bande der Referenz bei t=0. Je kleiner der Wert nach der Inkubation, desto stärker erfolgte ein Abbau des Proteinschmutzes.

Getestet wurden die in Beispiel 3 hergestellten Komplexe Ce-DO3A-C4-SO3⁻ (K1), Yb-DO3A-C4-SO3⁻ (K2), Sc-DO3A-C4-SO3⁻ (K3) und zum Vergleich der aus WO 2016/062784 A1 bekannte Komplex

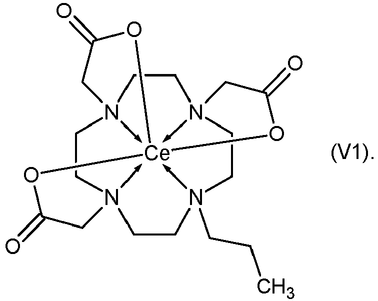

**Tabelle 1**

| Komplex | Intensität bei t=1h | Intensität bei t=5h | Intensität bei t=24h |
|---|---|---|---|
| - | 0,97 | 0,97 | 1,05 |
| V1 | 0,96 | 0,95 | 0,82 |
| K1 | 0,86 | 0,76 | 0,70 |
| K2 | 0,79 | 0,69 | 0,50 |
| K3 | 0,78 | 0,66 | 0,59 |

Man erkennt, dass die erfindungswesentlichen Komplexe die Proteinanschmutzung deutlich stärker abbauen als der bekannte Komplex mit dem Alkylsubstituenten am Stickstoffatom.

BSA wurde durch den in Beispiel 4 hergestellten Komplex (Komplex-Konzentration 2 mM) und einer Inkubationszeit von 16 Stunden unter ansonsten gleichen Bedingungen vollständig abgebaut.

## Patentansprüche

1. Geschirrspülmittel, insbesondere maschinelles Geschirrspülmittel, enthaltend einen Metallkomplex der allgemeinen Formel (III),
(A^{q+})ₚ[Mⁿ⁺L^{m-}](X^{o-})ᵣ (III)
in der q für 1 oder 2 und A⁺ für ein Kation, ausgewählt aus Alkalimetallkationen mit q = 1, ½Erdalkalikationen mit q = 2 und Ammoniumionen mit q = 1, steht, Mⁿ⁺ für ein Aluminiumion, ein Übergangsmetallion oder ein Lanthanoidmetallion steht, L für einen Liganden der Formel (I) 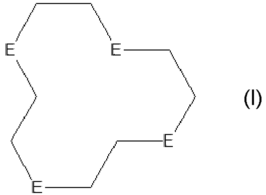 in der jedes E unabhängig voneinander für O oder NR¹ steht mit der Maßgabe, dass mindestens 1 E nicht O ist, jedes R¹ unabhängig voneinander für H, unsubstituiertes oder substituiertes, lineares oder verzweigtes C₁₋₂₂ Alkyl, unsubstituiertes oder substituiertes, lineares oder verzweigtes C₁₋₂₂ Heteroalkyl, unsubstituiertes oder substituiertes Aryl, unsubstituiertes oder substituiertes Heteroaryl, unsubstituiertes oder substituiertes, lineares oder verzweigtes C₂₋₂₀ Alkenyl, unsubstituiertes oder substituiertes, lineares oder verzweigtes C₂₋₂₀ Alkinyl, unsubstituiertes oder substituiertes, lineares oder verzweigtes C₂₋₂₀ Heteroalkenyl, unsubstituiertes oder substituiertes, lineares oder verzweigtes Alkylaryl, oder unsubstituiertes oder substituiertes, lineares oder verzweigtes Alkylheteroaryl steht mit der Maßgabe, dass mindestens 1 R¹ für ein substituiertes C₁₋₂₂ Alkyl der allgemeinen Formel (IIa) oder (IIc) steht, oder mit der Maßgabe, dass mindestens 1 R¹ für ein substituiertes C₁₋₂₂ Alkyl der allgemeinen Formel (IIIa) oder (IIIc) steht, 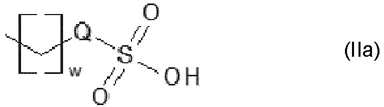 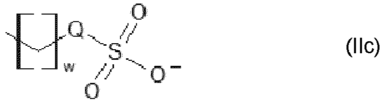 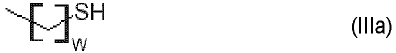 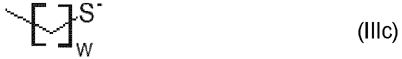 in denen Q für O oder CH₂, w für eine Zahl von 1 bis 22, q für 1 oder 2 und A⁺ für ein Kation, ausgewählt aus Alkalimetallkationen mit q = 1, ½Erdalkalikationen mit q = 2 und Ammoniumionen mit q = 1, steht, X^{o-} für ein Anion, ausgewählt aus F-, Cl⁻, Br, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, Acetat, Citrat, Formiat, Glutarat, Lactat, Malat, Malonat, Oxalat, Pyruvat, Tartrat, Methansulfonat, Methylsulfat, p-Toluolsulfat und Succinat, steht, n für eine Zahl von 1 bis 5 steht, m für eine Zahl von 0 bis 4 und o für eine Zahl von 1 bis 3 steht sowie p und r unabhängig voneinander für eine Zahl von 0 und 7 stehen mit der Maßgabe, dass die Summe aus n und dem Produkt aus p und q gleich der Summe aus m und dem Produkt aus r und o ist.

2. Verwendung eines Metallkomplexes der allgemeinen Formel (III),
(A^{q+})ₚ[Mⁿ⁺L^{m-}](X^{o-})ᵣ (III)
in der q für 1 oder 2 und A⁺ für ein Kation, ausgewählt aus Alkalimetallkationen mit q = 1, ½Erdalkalikationen mit q = 2 und Ammoniumionen mit q = 1, steht, Mⁿ⁺ für ein Aluminiumion, ein Übergangsmetallion oder ein Lanthanoidmetallion steht, L für einen Liganden der Formel (I) 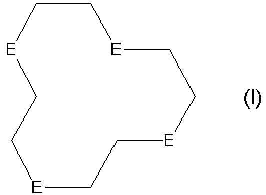 in der jedes E unabhängig voneinander für O oder NR¹ steht mit der Maßgabe, dass mindestens 1 E nicht O ist, jedes R¹ unabhängig voneinander für H, unsubstituiertes oder substituiertes, lineares oder verzweigtes C₁₋₂₂ Alkyl, unsubstituiertes oder substituiertes, lineares oder verzweigtes C₁₋₂₂ Heteroalkyl, unsubstituiertes oder substituiertes Aryl, unsubstituiertes oder substituiertes Heteroaryl, unsubstituiertes oder substituiertes, lineares oder verzweigtes C₂₋₂₀ Alkenyl, unsubstituiertes oder substituiertes, lineares oder verzweigtes C₂₋₂₀ Alkinyl, unsubstituiertes oder substituiertes, lineares oder verzweigtes C₂₋₂₀ Heteroalkenyl, unsubstituiertes oder substituiertes, lineares oder verzweigtes Alkylaryl, oder unsubstituiertes oder substituiertes, lineares oder verzweigtes Alkylheteroaryl steht mit der Maßgabe, dass mindestens 1 R¹ für ein substituiertes C₁₋₂₂ Alkyl der allgemeinen Formel (IIa) oder (IIc) steht, oder mit der Maßgabe, dass mindestens 1 R¹ für ein substituiertes C₁₋₂₂ Alkyl der allgemeinen Formel (IIIa) oder (IIIc) steht, 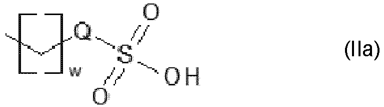 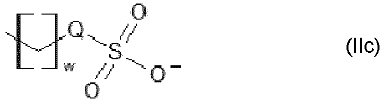 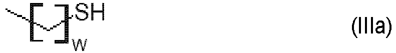 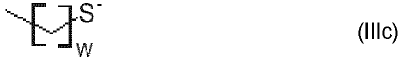 in denen Q für O oder CH₂, w für eine Zahl von 1 bis 22, q für 1 oder 2 und A⁺ für ein Kation, ausgewählt aus Alkalimetallkationen mit q = 1, ½Erdalkalikationen mit q = 2 und Ammoniumionen mit q = 1, steht, X^{o-} für ein Anion, ausgewählt aus F⁻, Cl⁻, Br⁻, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, Acetat, Citrat, Formiat, Glutarat, Lactat, Malat, Malonat, Oxalat, Pyruvat, Tartrat, Methansulfonat, Methylsulfat, p-Toluolsulfat und Succinat, steht, n für eine Zahl von 1 bis 5 steht, m für eine Zahl von 0 bis 4 und o für eine Zahl von 1 bis 3 steht sowie p und r unabhängig voneinander für eine Zahl von 0 und 7 stehen mit der Maßgabe, dass die Summe aus n und dem Produkt aus p und q gleich der Summe aus m und dem Produkt aus r und o ist, oder eines Geschirrspülmittels, das einen derartigen Komplex enthält, in einem maschinellen Geschirrspülverfahren.

3. Maschinelles Geschirrspülverfahren, bei dem ein Metallkomplex der allgemeinen Formel (III),
(A^{q+})ₚ[Mⁿ+L^{m-}](X^{o-})ᵣ (III)
in der q für 1 oder 2 und A⁺ für ein Kation, ausgewählt aus Alkalimetallkationen mit q = 1, ½Erdalkalikationen mit q = 2 und Ammoniumionen mit q = 1, steht, Mⁿ⁺ für ein Aluminiumion, ein Übergangsmetallion oder ein Lanthanoidmetallion steht, L für einen Liganden der Formel (I) 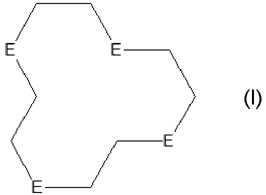 in der jedes E unabhängig voneinander für O oder NR¹ steht mit der Maßgabe, dass mindestens 1 E nicht O ist, jedes R¹ unabhängig voneinander für H, unsubstituiertes oder substituiertes, lineares oder verzweigtes C₁₋₂₂ Alkyl, unsubstituiertes oder substituiertes, lineares oder verzweigtes C₁₋₂₂ Heteroalkyl, unsubstituiertes oder substituiertes Aryl, unsubstituiertes oder substituiertes Heteroaryl, unsubstituiertes oder substituiertes, lineares oder verzweigtes C₂₋₂₀ Alkenyl, unsubstituiertes oder substituiertes, lineares oder verzweigtes C₂₋₂₀ Alkinyl, unsubstituiertes oder substituiertes, lineares oder verzweigtes C₂₋₂₀ Heteroalkenyl, unsubstituiertes oder substituiertes, lineares oder verzweigtes Alkylaryl, oder unsubstituiertes oder substituiertes, lineares oder verzweigtes Alkylheteroaryl steht mit der Maßgabe, dass mindestens 1 R¹ für ein substituiertes C₁₋₂₂ Alkyl der allgemeinen Formel (IIa) oder (IIc) steht, oder mit der Maßgabe, dass mindestens 1 R¹ für ein substituiertes C₁₋₂₂ Alkyl der allgemeinen Formel (IIIa) oder (IIIc) steht, 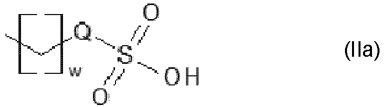 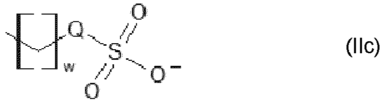 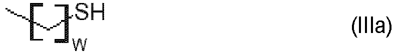 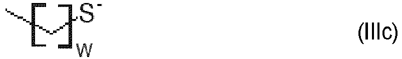 in denen Q für O oder CH₂, w für eine Zahl von 1 bis 22, q für 1 oder 2 und A⁺ für ein Kation, ausgewählt aus Alkalimetallkationen mit q = 1, ½Erdalkalikationen mit q = 2 und Ammoniumionen mit q = 1, steht, X^{o-} für ein Anion, ausgewählt aus F⁻, Cl⁻, Br, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, Acetat, Citrat, Formiat, Glutarat, Lactat, Malat, Malonat, Oxalat, Pyruvat, Tartrat, Methansulfonat, Methylsulfat, p-Toluolsulfat und Succinat, steht, n für eine Zahl von 1 bis 5 steht, m für eine Zahl von 0 bis 4 und o für eine Zahl von 1 bis 3 steht sowie p und r unabhängig voneinander für eine Zahl von 0 und 7 stehen mit der Maßgabe, dass die Summe aus n und dem Produkt aus p und q gleich der Summe aus m und dem Produkt aus r und o ist, oder ein Geschirrspülmittel, das einen derartigen Komplex enthält, zum Einsatz kommt.

4. Mittel nach Anspruch 1 oder Verwendung nach Anspruch 2 oder Verfahren nach Anspruch 3, **dadurch gekennzeichnet, dass** in der allgemeinen Formel (I) w für eine Zahl von 2 bis 20, insbesondere von 3 bis 18 steht.

5. Mittel, Verwendung oder Verfahren nach einem der voranstehenden Ansprüche, **dadurch gekennzeichnet, dass** in der allgemeinen Formel (I) genau 1 Gruppe R¹ der allgemeinen Formel (IIa) oder (IIb) oder (llc) entspricht.

6. Mittel, Verwendung oder Verfahren nach einem der voranstehenden Ansprüche, **dadurch gekennzeichnet, dass** in der allgemeinen Formel (I) die Gruppen R¹, die nicht der allgemeinen Formel (IIa), (IIb) oder (llc) entsprechen, aus 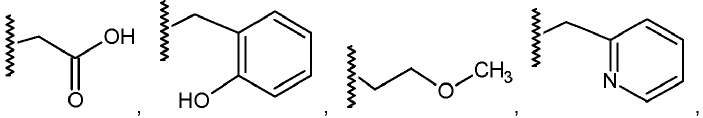 C₁₋₁₀ Alkyl, 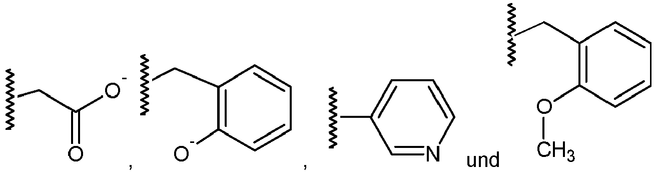 ausgewählt werden.

7. Verwendung oder Verfahren nach einem der voranstehenden Ansprüche, **dadurch gekennzeichnet, dass** Mⁿ⁺ aus Al³⁺, Ti⁴⁺, Y³⁺, Zr⁴⁺, La³⁺, Ce³⁺, Ce⁴⁺, Sc³⁺, Yb³⁺, Ta⁵⁺ und ihren Mischungen ausgewählt wird.

8. Mittel, Verwendung oder Verfahren nach einem der voranstehenden Ansprüche, **dadurch gekennzeichnet, dass** das Mittel 0,001 Gew.-% bis 10 Gew.-%, insbesondere 0,01 Gew.-% bis 3 Gew.-% des Metallkomplexes der Formel (III) enthält.

## Claims

1. A dishwashing detergent, in particular an automatic dishwashing detergent, containing a metal complex of the general formula (III),
(A^{q+})ₚ[Mⁿ⁺L^{m-}](X^{o-})ᵣ (III)
in which q represents 1 or 2 and A⁺ represents a cation selected from alkali metal cations where q=1, ½ alkaline earth cations where q=2, and ammonium ions where q=1, Mⁿ⁺ represents an aluminum ion, a transition metal ion or a lanthanide metal ion, L represents a ligand of the formula (I) 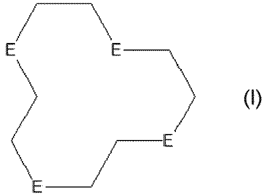 in which each E represents, independently of one another, O or NR¹ with the proviso that at least one E is not O, each R¹ represents, independently of one another, H, unsubstituted or substituted, linear or branched C₁₋₂₂ alkyl, unsubstituted or substituted, linear or branched C₁₋₂₂ heteroalkyl, unsubstituted or substituted aryl, unsubstituted or substituted heteroaryl, unsubstituted or substituted, linear or branched C₂₋₂₀ alkenyl, unsubstituted or substituted, linear or branched C₂₋₂₀ alkynyl, unsubstituted or substituted, linear or branched C₂₋₂₀ heteroalkenyl, unsubstituted or substituted, linear or branched alkylaryl, or unsubstituted or substituted, linear or branched alkyl heteroaryl, with the proviso that at least one R¹ represents a substituted C₁₋₂₂ alkyl of the general formula (IIa) or (IIc), or with the proviso that at least one R¹ represents a substituted C₁₋₂₂ alkyl of the general formula (IIIa) or (IIIc), 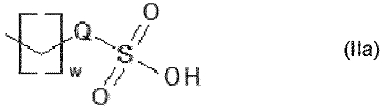 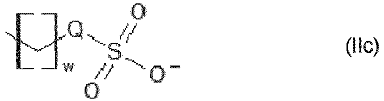 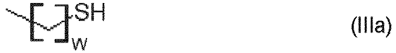 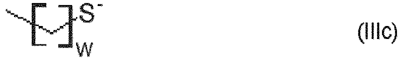 in which Q represents O or CH₂, w represents a number from 1 to 22, q represents 1 or 2 and A⁺ represents a cation selected from alkali metal cations where q=1, ½ alkaline earth cations where q=2, and ammonium ions where q=1, X^{o-} represents an anion selected from F⁻, Cl⁻, Br-, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acetate, citrate, formate, glutarate, lactate, malate, malonate, oxalate, pyruvate, tartrate, methane sulfonate, methyl sulfate, p-toluene sulfate and succinate, n represents a number from 1 to 5, m represents a number from 0 to 4 and o represents a number from 1 to 3, and p and r represent, independently of one another, a number from 0 and 7, with the proviso that the sum of n and the product of p and q is equal to the sum of m and the product of r and o.

2. The use of a metal complex of the general formula (III),
(A^{q+})ₚ[Mⁿ⁺L^{m-}](X^{o-})ᵣ (III)
in which q represents 1 or 2 and A⁺ represents a cation selected from alkali metal cations where q=1, ½ alkaline earth cations where q=2, and ammonium ions where q=1, Mⁿ⁺ represents an aluminum ion, a transition metal ion or a lanthanide metal ion, L represents a ligand of the formula (I) 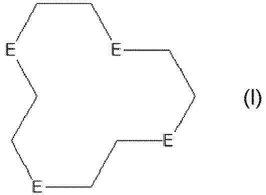 in which each E represents, independently of one another, O or NR¹ with the proviso that at least one E is not O, each R¹ represents, independently of one another, H, unsubstituted or substituted, linear or branched C₁₋₂₂ alkyl, unsubstituted or substituted, linear or branched C₁₋₂₂ heteroalkyl, unsubstituted or substituted aryl, unsubstituted or substituted heteroaryl, unsubstituted or substituted, linear or branched C₂₋₂₀ alkenyl, unsubstituted or substituted, linear or branched C₂₋₂₀ alkynyl, unsubstituted or substituted, linear or branched C₂₋₂₀ heteroalkenyl, unsubstituted or substituted, linear or branched alkylaryl, or unsubstituted or substituted, linear or branched alkyl heteroaryl, with the proviso that at least one R¹ represents a substituted C₁₋₂₂ alkyl of the general formula (IIa) or (IIc), or with the proviso that at least one R¹ represents a substituted C₁₋₂₂ alkyl of the general formula (IIIa) or (IIIc), 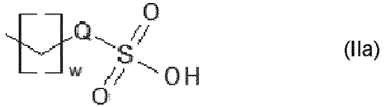 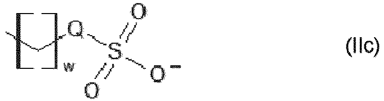 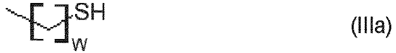 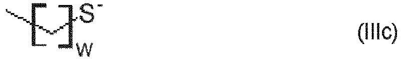 in which Q represents O or CH₂, w represents a number from 1 to 22, q represents 1 or 2 and A⁺ represents a cation selected from alkali metal cations where q=1, ½ alkaline earth cations where q=2, and ammonium ions where q=1, X^{o-} represents an anion selected from F⁻, Cl⁻, Br-, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acetate, citrate, formate, glutarate, lactate, malate, malonate, oxalate, pyruvate, tartrate, methane sulfonate, methyl sulfate, p-toluene sulfate and succinate, n represents a number from 1 to 5, m represents a number from 0 to 4 and o represents a number from 1 to 3, and p and r represent, independently of one another, a number from 0 and 7, with the proviso that the sum of n and the product of p and q is equal to the sum of m and the product of r and o, or a dishwashing detergent containing such a complex in an automatic dishwashing method.

3. An automatic dishwashing method in which a metal complex of the general formula (III),
(A^{q+})ₚ[Mⁿ⁺L^{m-}](X^{o-})ᵣ (III)
in which q represents 1 or 2 and A⁺ represents a cation selected from alkali metal cations where q=1, ½ alkaline earth cations where q=2, and ammonium ions where q=1, Mⁿ⁺ represents an aluminum ion, a transition metal ion or a lanthanide metal ion, L represents a ligand of the formula (I) 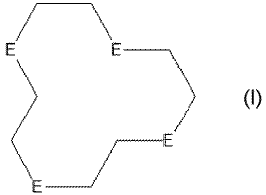 in which each E represents, independently of one another, O or NR¹ with the proviso that at least one E is not O, each R¹ represents, independently of one another, H, unsubstituted or substituted, linear or branched C₁₋₂₂ alkyl, unsubstituted or substituted, linear or branched C₁₋₂₂ heteroalkyl, unsubstituted or substituted aryl, unsubstituted or substituted heteroaryl, unsubstituted or substituted, linear or branched C₂₋₂₀ alkenyl, unsubstituted or substituted, linear or branched C₂₋₂₀ alkynyl, unsubstituted or substituted, linear or branched C₂₋₂₀ heteroalkenyl, unsubstituted or substituted, linear or branched alkylaryl, or unsubstituted or substituted, linear or branched alkyl heteroaryl, with the proviso that at least one R¹ represents a substituted C₁₋₂₂ alkyl of the general formula (IIa) or (IIc), or with the proviso that at least one R¹ represents a substituted C₁₋₂₂ alkyl of the general formula (IIIa) or (IIIc), 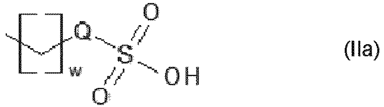 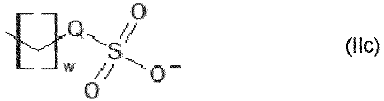 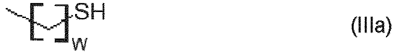 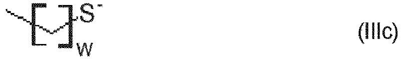 in which Q represents O or CH₂, w represents a number from 1 to 22, q represents 1 or 2 and A+ represents a cation selected from alkali metal cations where q=1, ½ alkaline earth cations where q=2, and ammonium ions where q=1, Xo- represents an anion selected from F⁻, Cl⁻, Br⁻, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acetate, citrate, formate, glutarate, lactate, malate, malonate, oxalate, pyruvate, tartrate, methane sulfonate, methyl sulfate, p-toluene sulfate and succinate, n represents a number from 1 to 5, m represents a number from 0 to 4 and o represents a number from 1 to 3, and p and r represent, independently of one another, a number from 0 and 7, with the proviso that the sum of n and the product of p and q is equal to the sum of m and the product of r and o, or a dishwashing detergent containing such a complex is used.

4. The detergent according to claim 1 or the use according to claim 2 or the method according to claim 3, **characterized in that** in the general formula (I), w represents a number from 2 to 20, in particular from 3 to 18.

5. The detergent, use or method according to one of the preceding claims, **characterized in that** in the general formula (I), exactly one group R¹ corresponds to the general formula (IIa) or (IIb) or (IIc).

6. The detergent, use or method according to one of the preceding claims, **characterized in that** in the general formula (I), the groups R¹ which do not correspond to the general formula (IIa), (IIb) or (IIc) are selected from 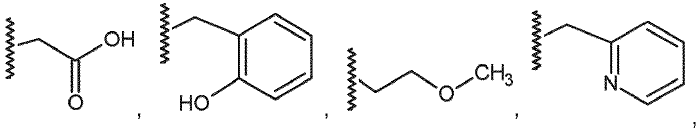 C₁₋₁₀ Alkyl,

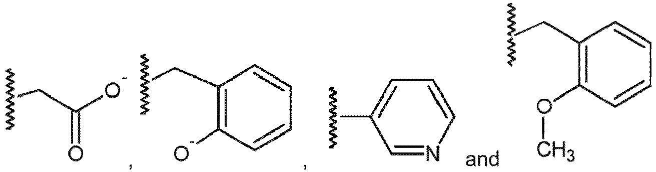

7. The use or method according to one of the preceding claims, **characterized in that** Mⁿ⁺ is selected from Al³⁺, Ti⁴⁺, Y³⁺, Zr⁴⁺, La³⁺, Ce³⁺, Ce⁴⁺, Sc³⁺, Yb³⁺, Ta⁵⁺ and mixtures thereof.

8. The detergent, use or method according to one of the preceding claims, **characterized in that** the detergent contains 0.001 wt.% to 10 wt.%, in particular 0.01 wt.% to 3 wt.% of the metal complex of the formula (III).

## Revendications

1. Agent de lavage pour vaisselle, en particulier agent de lavage pour vaisselle en machine, contenant un complexe métallique de formule générale (III),
(A^{q+})ₚ[Mⁿ⁺L^{m-}](X^{o-})ᵣ (III)
dans laquelle q représente 1 ou 2 et A⁺ représente un cation choisi parmi les cations de métaux alcalins avec q = 1, les ½ cations alcalino-terreux avec q = 2 et les ions ammonium avec q = 1, Mⁿ⁺ représente un ion aluminium, un ion de métal de transition ou un ion de métal lanthanide, L représente un ligand de formule (I) 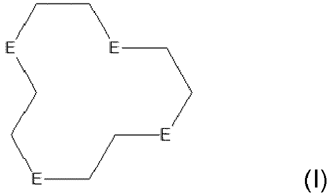 dans laquelle chaque E représente indépendamment les uns des autres O ou NR¹, à condition qu'au moins 1 E ne représente pas O, chaque R¹ représente indépendamment les uns des autres H, un alkyle en C₁₋₂₂ linéaire ou ramifié, non substitué ou substitué, un hétéroalkyle en C₁₋₂₂ linéaire ou ramifié, non substitué ou substitué, un aryle non substitué ou substitué, un hétéroaryle non substitué ou substitué, un alcényle en C₂₋₂₀ linéaire ou ramifié, non substitué ou substitué, un alcynyle en C₂₋₂₀ linéaire ou ramifié, non substitué ou substitué, un hétéroalcényle en C₂₋₂₀ linéaire ou ramifié, non substitué ou substitué, un alkylaryle linéaire ou ramifié, non substitué ou substitué, ou un alkylhétéroaryle linéaire ou ramifié, non substitué ou substitué, à condition qu'au moins 1 R¹ représente un alkyle en C₁₋₂₂ substitué de formule générale (IIa) ou (IIc), ou à condition qu'au moins 1 R¹ représente un alkyle en C₁₋₂₂ substitué de formule générale (IIIa) ou (IIIc), 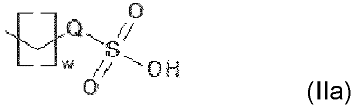 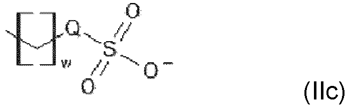 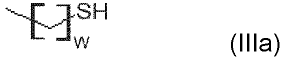 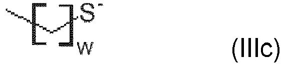 dans lesquelles Q représente O ou CH₂, w représente un nombre de 1 à 22, q représente 1 ou 2 et A⁺ représente un cation choisi parmi les cations de métaux alcalins avec q = 1, les ½ cations alcalino-terreux avec q = 2 et les ions ammonium avec q = 1, X^{o-} représente un anion choisi parmi F⁻, Cl⁻, Br-, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acétate, citrate, formiate, glutarate, lactate, malate, malonate, oxalate, pyruvate, tartrate, méthanesulfonate, méthylsulfate, p-toluènesulfate et succinate, n représente un nombre de 1 à 5, m représente un nombre de 0 à 4 et o représente un nombre de 1 à 3 et p et r représentent indépendamment l'un de l'autre un nombre de 0 à 7, à condition que la somme de n et du produit de p et q soit égale à la somme de m et du produit de r et o.

2. Utilisation d'un complexe métallique de formule générale (III),
(A^{q+})ₚ[Mⁿ⁺L^{m-}](X^{o-})ᵣ (III)
dans laquelle q représente 1 ou 2 et A⁺ représente un cation choisi parmi les cations de métaux alcalins avec q = 1, les ½ cations alcalino-terreux avec q = 2 et les ions ammonium avec q = 1, Mⁿ⁺ représente un ion aluminium, un ion de métal de transition ou un ion de métal lanthanide, L représente un ligand de formule (I) 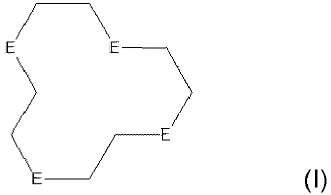 dans laquelle chaque E représente indépendamment les uns des autres O ou NR¹, à condition qu'au moins 1 E ne représente pas O, chaque R¹ représente indépendamment les uns des autres H, un alkyle en C₁₋₂₂ linéaire ou ramifié, non substitué ou substitué, un hétéroalkyle en C₁₋₂₂ linéaire ou ramifié, non substitué ou substitué, un aryle non substitué ou substitué, un hétéroaryle non substitué ou substitué, un alcényle en C₂₋₂₀ linéaire ou ramifié, non substitué ou substitué, un alcynyle en C₂₋₂₀ linéaire ou ramifié, non substitué ou substitué, un hétéroalcényle en C₂₋₂₀ linéaire ou ramifié, non substitué ou substitué, un alkylaryle linéaire ou ramifié, non substitué ou substitué, ou un alkylhétéroaryle linéaire ou ramifié, non substitué ou substitué, à condition qu'au moins 1 R¹ représente un alkyle en C₁₋₂₂ substitué de formule générale (IIa) ou (IIc), ou à condition qu'au moins 1 R¹ représente un alkyle en C₁₋₂₂ substitué de formule générale (IIIa) ou (IIIc), 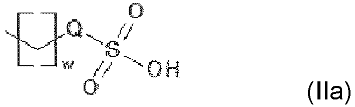 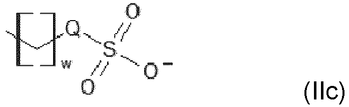 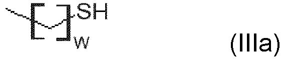 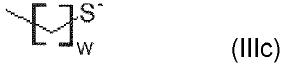 dans lesquelles Q représente O ou CH₂, w représente un nombre de 1 à 22, q représente 1 ou 2 et A⁺ représente un cation choisi parmi les cations de métaux alcalins avec q = 1, les ½ cations alcalino-terreux avec q = 2 et les ions ammonium avec q = 1, X^{o-} représente un anion choisi parmi F⁻, Cl⁻, Br-, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acétate, citrate, formiate, glutarate, lactate, malate, malonate, oxalate, pyruvate, tartrate, méthanesulfonate, méthylsulfate, p-toluènesulfate et succinate, n représente un nombre de 1 à 5, m représente un nombre de 0 à 4 et o représente un nombre de 1 à 3 et p et r représentent indépendamment l'un de l'autre un nombre de 0 à 7, à condition que la somme de n et du produit de p et q soit égale à la somme de m et du produit de r et o, ou d'un agent de lavage pour vaisselle contenant un tel complexe dans un procédé de lavage de vaisselle en machine.

3. Procédé de lavage de vaisselle en machine, dans lequel est utilisé un complexe métallique de formule générale (III),
(A^{q+})ₚ[Mⁿ⁺L^{m-}](X^{o-})ᵣ (III)
dans laquelle q représente 1 ou 2 et A⁺ représente un cation choisi parmi les cations de métaux alcalins avec q = 1, les ½ cations alcalino-terreux avec q = 2 et les ions ammonium avec q = 1, Mⁿ⁺ représente un ion aluminium, un ion de métal de transition ou un ion de métal lanthanide, L représente un ligand de formule (I) 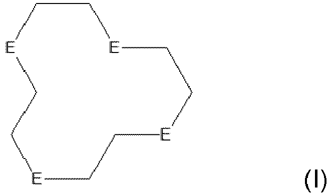 dans laquelle chaque E représente indépendamment les uns des autres O ou NR¹, à condition qu'au moins 1 E ne représente pas O, chaque R¹ représente indépendamment les uns des autres H, un alkyle en C₁₋₂₂ linéaire ou ramifié, non substitué ou substitué, un hétéroalkyle en C₁₋₂₂ linéaire ou ramifié, non substitué ou substitué, un aryle non substitué ou substitué, un hétéroaryle non substitué ou substitué, un alcényle en C₂₋₂₀ linéaire ou ramifié, non substitué ou substitué, un alcynyle en C₂₋₂₀ linéaire ou ramifié, non substitué ou substitué, un hétéroalcényle en C₂₋₂₀ linéaire ou ramifié, non substitué ou substitué, un alkylaryle linéaire ou ramifié, non substitué ou substitué, ou un alkylhétéroaryle linéaire ou ramifié, non substitué ou substitué, à condition qu'au moins 1 R¹ représente un alkyle en C₁₋₂₂ substitué de formule générale (IIa) ou (IIc), ou à condition qu'au moins 1 R¹ représente un alkyle en C₁₋₂₂ substitué de formule générale (IIIa) ou (IIIc), 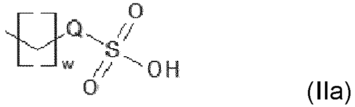 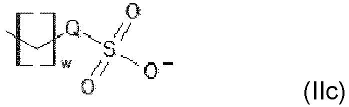 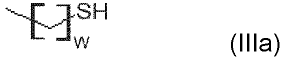 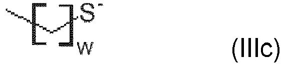 dans lesquelles Q représente O ou CH₂, w représente un nombre de 1 à 22, q représente 1 ou 2 et A⁺ représente un cation choisi parmi les cations de métaux alcalins avec q = 1, les ½ cations alcalino-terreux avec q = 2 et les ions ammonium avec q = 1, X^{o-} représente un anion choisi parmi F⁻, Cl⁻, Br-, I⁻, OH⁻, HSO₃⁻, SO₃²⁻, SO₄²⁻, HSO₄⁻, NO₂⁻, NO₃⁻, PO₄³⁻, HPO₄²⁻, H₂PO₄⁻, BF₄⁻, PF₆⁻, ClO₄⁻, acétate, citrate, formiate, glutarate, lactate, malate, malonate, oxalate, pyruvate, tartrate, méthanesulfonate, méthylsulfate, p-toluènesulfate et succinate, n représente un nombre de 1 à 5, m représente un nombre de 0 à 4 et o représente un nombre de 1 à 3 et p et r représentent indépendamment l'un de l'autre un nombre de 0 à 7, à condition que la somme de n et du produit de p et q soit égale à la somme de m et du produit de r et o, ou un agent de lavage pour vaisselle contenant un tel complexe.

4. Agent selon la revendication 1 ou utilisation selon la revendication 2 ou procédé selon la revendication 3, **caractérisé en ce que,** dans la formule générale (I), w représente un nombre de 2 à 20, en particulier de 3 à 18.

5. Agent, utilisation ou procédé selon l'une des revendications précédentes, **caractérisé en ce que,** dans la formule générale (I), exactement 1 groupe R¹ correspond à la formule générale (IIa) ou (IIb) ou (IIc).

6. Agent, utilisation ou procédé selon l'une des revendications précédentes, **caractérisé en ce que,** dans la formule générale (I), les groupes R¹ qui ne correspondent pas à la formule générale (IIa), (IIb) ou (IIc) sont choisis parmi 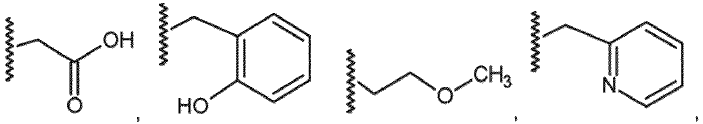 C₁₋₁₀ Alkyl,

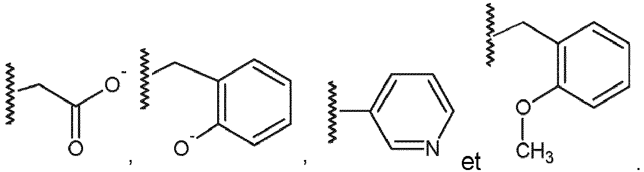

7. Utilisation ou procédé selon l'une des revendications précédentes, **caractérisé en ce que** Mⁿ⁺ est choisi parmi Al³⁺, Ti⁴⁺, Y³⁺, Zr⁴⁺, La³⁺, Ce³⁺, Ce⁴⁺, Sc³⁺, Yb³⁺, Ta⁵⁺ et leurs mélanges.

8. Agent, utilisation ou procédé selon l'une des revendications précédentes, **caractérisé en ce que** l'agent contient 0,001 % en poids à 10 % en poids, en particulier 0,01 % en poids à 3 % en poids, du complexe métallique de formule (III).
